(19) Europäisches Patentamt
European Patent Office
Office européen des brevets

(11) **EP 4 389 156 A1**

(12) **EUROPEAN PATENT APPLICATION**

(43) Date of publication:
**26.06.2024 Bulletin 2024/26**

(21) Application number: **22215651.5**

(22) Date of filing: **21.12.2022**

(51) International Patent Classification (IPC):
***A61K 51/04*** *(2006.01)* ***A61P 35/00*** *(2006.01)*
***A61K 103/10*** *(2006.01)*

(52) Cooperative Patent Classification (CPC):
**A61K 51/0497; A61K 51/0402; A61P 35/00**

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**KH MA MD TN**

(71) Applicant: **Technische Universität München**
**80333 München (DE)**

(72) Inventors:
- **WESTER, Hans-Jürgen**
  **85301 Schweitenkirchen (DE)**
- **KUNERT, Jan-Philip**
  **86154 Augsburg (DE)**

(74) Representative: **Vossius & Partner**
**Patentanwälte Rechtsanwälte mbB**
**Siebertstraße 3**
**81675 München (DE)**

(54) **RADIOLABELED PSMA LIGAND COMPOUNDS AND PRECURSORS THEREOF**

(57) Provided is a PSMA-targeted ligand compound, which is a compound of formula (1) or a salt thereof:

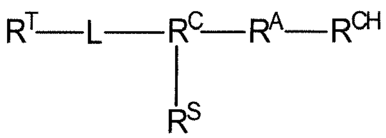

(1)

wherein $R^T$ is a PSMA binding group; L is a linking group; $R^C$ is a trivalent coupling group; $R^S$ is a silicon-containing moiety of the formula -C(O)-$R^{S3}$-Si$R^{S1}R^{S2}$OH, wherein $R^{S1}$ and $R^{S2}$ are independently selected from C3-C10 alkyl, and are preferably tert-butyl, and $R^{S3}$ is a group comprising a 6 membered aromatic ring, and is preferably benzenediyl; $R^A$ is an amino acid unit; and $R^{CH}$ is selected from (i) a branched-chain, acyclic chelating moiety having 4 amino groups, and (ii) a chelate moiety, wherein a radioisotope selected from $^{99m}$Tc, $^{94m}$Tc, $^{186}$Re and $^{188}$Re is chelated by the branched-chain, acyclic chelating moiety having 4 amino groups. The PSMA-targeted ligand compound in accordance with the invention is suitable for radiopharmaceutical applications, such as radioguided surgery.

EP 4 389 156 A1

## Description

The invention relates to compounds which are suitable as PSMA binding ligand compounds which can be radiolabeled and which are suitable for application as radiotracers, e.g. in the context of radiodiagnosis or radioguided surgery, or for radiotherapeutic applications.

Throughout the last decade, radiopharmaceuticals targeting glutamate carboxypeptidase II (GCPII) which is also referred to as prostate specific membrane antigen (PSMA), have become an integral part in clinical management of prostate cancer (PCa) [1, 2]. Almost in parallel with the clinical success of diagnostic tracers such as [$^{68}$Ga]Ga-PSMA-11 [3] and [$^{18}$F]DCFPyL [4] for positron emission tomography (PET) or [$^{99m}$Tc]Tc-MIP-1404 [5] for single-photon emission computed tomography (SPECT) imaging, several therapeutic compounds entered the stage, among them $^{177}$Lu-labeled PSMA-I&T and PSMA-617 [6, 7]. In addition, several distinct therapeutic approaches have found their way into preclinical and clinical research, among them long-acting albumin-binding PSMA-ligands [8, 9], targeted alpha therapy [10] or so-called tandem therapy combining [$^{225}$Ac]Ac- and [$^{177}$Lu]Lu-PSMA-617 [11]. The recent approval of [$^{177}$Lu]Lu-PSMA-617 (Pluvicto™, Novartis) for RLT of metastatic castration-resistant PCa (mCRPC) represents a milestone for nuclear medicine, broadens the armamentarium of oncologists, and might pave the way for further approved targeted therapeutic radiopharmaceuticals.

Radioguided surgery (RGS) is another therapeutic intervention successfully harnessing the potential of radioactive PSMA-targeted probes [12]. Patients with early biochemical recurrence after RP that show only regional pelvic lymph node metastases (LNM) in PSMA-PET imaging can benefit from radioguided salvage lymph node dissection (sLND) to delay disease progression and future systemic treatment [13]. In contrast to conventional sLND, a $\gamma$-emitting PSMA-targeted radioligand is intravenously injected up to 24 h prior to surgery. With the help of a $\gamma$-probe, localization and resection of metastatic lymph nodes are facilitated during surgery, which is especially useful in the case of small or atypically localized lesions. Furthermore, resected tissue can be identified instantly by *ex vivo* $\gamma$-probe measurements to confirm the successful removal of tumor-infested tissue. After initial proof-of-concept with [$^{111}$In]In-PSMA-I&T [14], PSMA-RGS has been carried out with [$^{99m}$Tc]Tc-PSMA-I&S [15], owing to its similar performance *in vivo* and more favorable radiation properties, the more common allowance to work with $^{99m}$Tc-tracers in surgery rooms, lower costs and higher availability of [$^{99m}$Tc]$TcO_4^-$ compared to [$^{111}$In]$InCl_3$. A recent study with 121 patients by *Horn et al.* described the successful removal of preoperatively identified lesions in 99% of patients and a complete biochemical response in 66% of patients [16], which confirms results reported earlier by *Maurer et al.* for a smaller group of patients [13]. In the latter study, even additional lesions not previously detected on PSMA-PET could be removed, which highlights the potential of RGS for sLND [13]. However, prospective clinical trials such as the TRACE study (NCT03857113) are required to accurately assess the long-term outcome and added benefit of RGS for PCa patients.

Whether conventional or radioguided, the main limitation of sLND procedures is constituted in the incomplete resection of metastatic lesions. Even though state of the art PSMA radio-guidance using [$^{99m}$Tc]Tc-PSMA-I&S showed superior short-term efficacy in terms of biochemical response in a prospective study by *Knipper et al.* [17], the still insufficient sensitivity with regard to micro-metastatic lesions seems to be a major reason for recurrent disease [13, 18]. Technological developments like robot-assisted laparoscopic surgery implying drop-in or click-on $\gamma$-probes [19] and more differentiated criteria for patient selection [20] can certainly move the application forward. However, on a radiopharmaceutical level we identified a pending need of improvement, namely a radiotracer providing higher contrast and lower background signal than currently applied [$^{99m}$Tc]Tc-PSMA-I&S. its comparably slow whole-body clearance and partial hepatobiliary excretion is caused by high plasma protein binding (PPB) of 94% and reduced hydrophilicity compared to DOTA-based PSMA-radiochelated tracers [15]. As a result, unspecific background signal hampers both contrast in early SPECT-imaging and accurate detection during surgery [21, 22]. Thus, often [$^{99m}$Tc]Tc-PSMA-I&S is not used to reliably identify LNMs *in situ* during surgery, but rather to confirm PSMA-positivity of resected tissue *ex vivo*. However, technically a more accurate lesion detection even in the surgical field should be feasible if TBR are sufficiently high, and thus, even smaller lesions might become detectable with an appropriately performing radiotracer. In spite of the known limitations of [$^{99m}$Tc]Tc-PSMA-I&S, to our knowledge, no dedicated optimization of a $^{99m}$Tc-labeled PSMA-ligand intended for RGS application has been performed so far.

To address this medical need, the invention provides a novel series of radiolabeled PSMA-targeted ligand compounds and their precursors with improved pharmacokinetic behavior, which are suitable for application as radiotracers (or radioactive targeted probes), e.g. for radiodiagnosis or in the context of RGS or for radiotherapeutic applications. The optimized ligand structure was conceptually designed and comprises a common PSMA-inhibitor motif derived from highly potent radiohybrid PSMA diagnostics [23] and therapeutics [24], a tetraamine chelator for reliable complexation of a suitable radioisotope, such as technetium-99m, and a variable amino acid for the modulation of pharmacokinetic properties of the peptide. Such modifications have brought forth interesting PSMA radioligands for manifold applications [25-27].

The PSMA-targeted ligand compounds in accordance with the invention are compounds of formula (1) or a salt thereof:

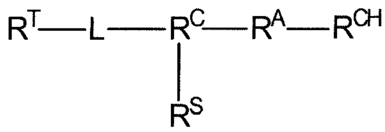

(1)

wherein

- $R^T$ is a PSMA binding group;
- L is a linking group;
- $R^C$ is a trivalent coupling group;
- $R^S$ is a silicon-containing moiety of the formula $-C(O)-R^{S3}-SiR^{S1}R^{S2}OH$, wherein $R^{S1}$ and $R^{S2}$ are independently selected from C3-C10 alkyl, and are preferably *tert*-butyl, and $R^{S3}$ is a group comprising a 6 membered aromatic ring, and is preferably benzenediyl;
- $R^A$ is an amino acid unit; and
- $R^{CH}$ is selected from

  (i) a branched-chain, acyclic chelating moiety having 4 amino groups, and

  (ii) a chelate moiety, wherein a radioisotope selected from $^{99m}Tc$, $^{94m}Tc$, $^{186}Re$ and $^{188}Re$ is chelated by the branched-chain, acyclic chelating moiety having 4 amino groups.

It has been found that the compounds in accordance with the invention combine a pronounced capability of binding as inhibitors to PSMA with general advantages compared to PSMA-I&S, such as reduced lipophilicity and reduced plasma protein binding (PPB). Thus, the compounds in accordance with the invention exhibit similar or even improved *in vitro* characteristics, while showing increased tumor-to-organ ratios in a variety of organs and in particular an increased tumor-to-kidney ratio, which is a critical parameter for radioligand applications in the diagnosis and the treatment of prostate cancer.

Thus, as a further aspect, the invention relates to compositions comprising a compound in accordance with the invention, e.g. a therapeutic or diagnostic composition. Such a composition is suitable for the treatment and/or diagnosis of a disease which is associated with the overexpression of PSMA, such as prostate cancer.

As explained above, the compounds in accordance with the invention encompass compounds of formula (1). Moreover, salts, typically pharmaceutically acceptable salts, of the compounds of formula (1) are encompassed by the present invention. Thus, unless indicated to the contrary, any reference to a compound in accordance with the invention herein encompasses the compounds of formula (1) (and the preferred embodiments of these formulae disclosed herein), and the salts thereof. Likewise, any racemates, enantiomers, or diastereomers of any chiral compounds of formula (1) and their salts are encompassed, unless a specific stereochemistry of the compound under consideration is indicated in a specific context. Due to their capability of binding to and of acting as a ligand for PSMA, the compounds in accordance with the invention may also be referred to as PSMA-targeted ligand compounds in accordance with the invention, as PSMA-binding compounds in accordance with the invention, or briefly as ligand compounds in accordance with the invention.

In the following, a further description will be provided of the compounds of formula (1) and their salts, and of preferred embodiments thereof. In line with the above explanation, it will be understood that groups discussed in this context which may provide a salt form of the compounds of formula (1), such as acidic groups which can be deprotonated or basic groups which can be protonated, can be contained in the compounds in accordance with the invention in an ionic (e.g. protonated or deprotonated) form, even if this ionic form is not specifically illustrated for the concerned group herein.

$R^T$ in formula (1) represents a PSMA binding group. Due to the capability of binding to PSMA, it allows the compounds in accordance with the invention to function as PSMA-targeting ligand compounds. A variety of PSMA binding groups is known in the art and available to the skilled person for this purpose.

In formula (1), $R^T$ is preferably a group of the following structure (T-1):

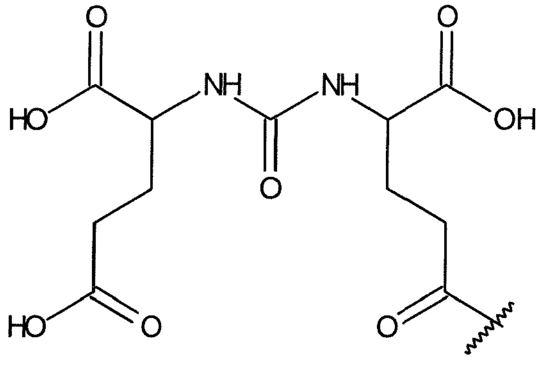

(T-1)

wherein the waved line marks the bond which attaches the group $R^T$ to L in formula (1). More preferably, $R^T$ is a group of the following structure (T-2):

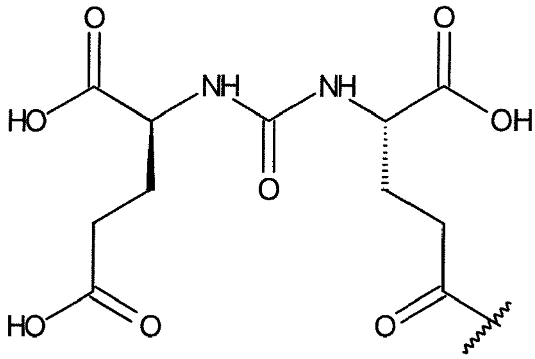

(T-2)

wherein the waved line marks the bond which attaches the group $R^T$ to L in formula (1).

As will be understood by the skilled person, one or more of the carboxylic acid groups shown in the above formulae may be deprotonated, depending on the conditions under which the compounds in accordance with the invention are kept, e.g. the pH of a solution thereof. The negative charge of a deprotonated carboxylic acid group may be balanced, e.g., by a positive charge carried by another group in a compound in accordance with the invention, or by a cationic counterion, e.g., as discussed below with regard to salt forms of the compounds in accordance with the invention.

In line with the above, the compound of formula (1) is preferably a compound of formula (1A):

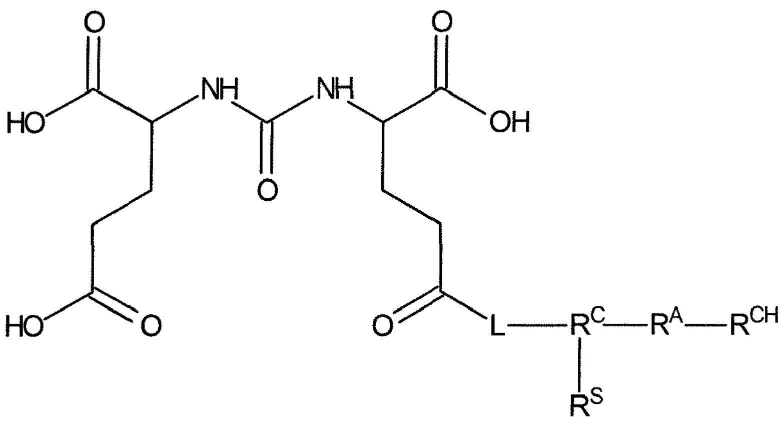

(1A),

wherein L, $R^C$, $R^S$, $R^A$ and $R^{CH}$ are as defined herein, including any preferred definitions thereof.

Still more preferably, the compound of formula (1) is a compound of formula (1AA):

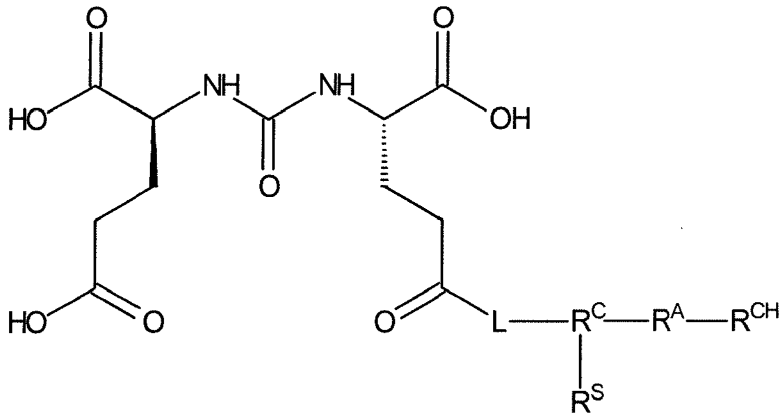

(1AA),

wherein L, $R^C$, $R^S$, $R^A$ and $R^{CH}$ are as defined herein, including any preferred definitions thereof.

It should be understood that any reference to formula (1) herein, unless indicated to the contrary, applies likewise to formulae (1A) and (1AA) as preferred variants thereof.

The group L in formula (1) represent a linking group. As illustrated in formula (1), L is a divalent group which is attached via a first covalent bond to $R^T$ and via a second covalent bond to the coupling group $R^C$. Thus, L typically contains a first functional group at a first terminus to which $R^T$ is attached and which is suitable to form an amide bond -C(O)-NH- or an alkylated amide bond -C(O)-NR-, preferably an amide bond, with a complementary group contained in $R^T$. Preferably, this first functional group in L is a group -NH-. Likewise, L typically contains a second functional group at a second terminus to which $R^C$ is attached and which is suitable to form an amide bond or an alkylated amide bond, preferably an amide bond, with a complementary group contained in $R^C$. Preferably, this second functional group in L is also a group -NH-.

If reference is made herein to an alkylated amide bond -C(O)-NR- (or -NR-C(O)-), the group R of the alkylated amide bond, also in the following, is a C1-C6 alkyl group, and is preferably methyl.

In a preferred embodiment, L represents an oligoamide residue with a first and a second terminus providing the functional groups discussed above, i.e. preferably a fist and a second terminus providing a group -NH-. The oligoamide residue preferably comprises 2 to 6, more preferably 2 to 4, and still more preferably 3 subunits. As implied by the designation as an oligoamide residue, adjacent subunits in the oligoamide residue are linked to each other via an amide bond -C(O)-NH- or an alkylated amide bond -C(O)-NR-, preferably by an amide bond. Preferably, the backbone of the oligoamide unit which extends from the first to the second terminus comprises 6 to 20, more preferably 8 to 18, and still more preferably 8 to 16 carbon atoms, not including carbon atoms in optional substituent groups attached to the backbone.

As an example of a linking group L, the following group (L-1) can be illustrated:

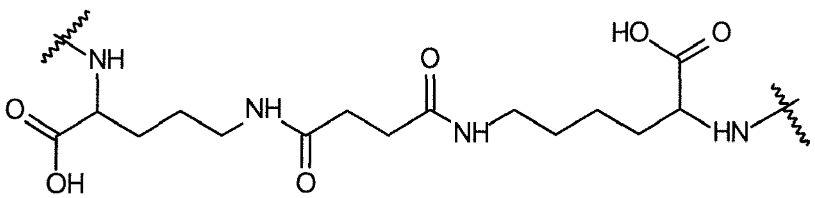

(L-1)

wherein the waved lines mark bonds which are formed by L to $R^T$ and $R^C$, respectively.

As will be understood by the skilled person, one or more of the carboxylic acid groups shown in the above formula may be deprotonated, depending on the conditions under which the compounds in accordance with the invention are kept, e.g. the pH of a solution thereof. The negative charge of a deprotonated carboxylic acid group may be balanced, e.g., by a positive charge carried by another group in a compound in accordance with the invention, or by a cationic counterion, e.g., as discussed below with regard to salt forms of the compounds in accordance with the invention.

$R^C$ in formula (1) is a trivalent coupling group which serves to couple the branch $R^T$-L-, the branch -$R^A$-$R^{CH}$ and the group -$R^S$ as shown in formula (1). Thus, $R^C$ typically contains a functional group at its terminus to which L is attached, which is suitable to form an amide bond (-C(O)-NH-) or an alkylated amide bond (-C(O)-NR-), preferably an amide bond, with a complementary group contained in L. Preferably, this functional group in $R^C$ is a group -C(O)-. Likewise, $R^C$ typically contains a functional group, either at a terminus or in a side chain to which $R^A$ is attached, which is suitable to form an amide bond, or an alkylated amide bond, preferably an amide bond, with a complementary group contained in $R^A$. Preferably, this coupling group in $R^C$ is a group -NH-. At the terminus to which $R^S$ is attached, $R^C$

typically contains a functional group, either at a terminus or in a side chain to which $R^S$ is attached, which is suitable to form an amide bond or an alkylated amide bond, preferably an amide bond, with the complementary group -C(O)- contained in $R^S$. Preferably, this coupling group in $R^C$ is a group -NH-.

It is preferred that $R^C$ is a trivalent amino acid unit. More preferably $R^C$ is a trivalent amino acid unit which can be derived from an amino acid comprising, together with the carboxylic acid group and the amino group, a side chain carrying a further functional group selected form a carboxylic acid group and an amino group. More preferably, $R^C$ is selected from a 2,3-diaminopropionic acid (Dap) unit, 2,4-diaminobutanoic acid (Dab) unit, ornithine (Orn) unit and a lysine (Lys) unit, most preferably a Dap unit. As will be understood by the skilled reader, the amino acid unit is a unit derived from the respective amino acid by using its functional groups to provide a bond, preferably an amide bond, to an adjacent group to which the amino acid unit is attached. With respect to their stereochemistry, these amino acid units are preferably D-amino acid units.

The trivalent amino acid unit is preferably attached in the compound of the invention by three amide bonds. Moreover, it is more preferred that the amino acid unit is oriented to provide a -C(O)- functional group attached to L so that an amide bond is formed by the amino acid unit $R^C$ with L, a -NH- functional group attached to $R^A$ so that an amide bond is formed by the amino acid unit $R^C$ with $R^A$, and a -NH- functional group attached to $R^S$, so that an amide bond is formed by the amino acid unit $R^C$ with $R^S$.

$R^S$ in formula (1) is a silicon-containing moiety of the formula -C(O)-$R^{S3}$-Si$R^{S1}R^{S2}$OH, which can also be illustrated by formula (S-1)

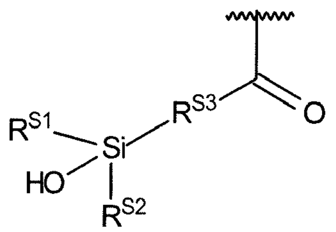

(S-1),

wherein $R^{S1}$ and $R^{S2}$ are independently selected from C3-C10 alkyl, and are preferably *tert*-butyl, and $R^{S3}$ is a group comprising a 6 membered aromatic ring. Preferably, $R^{S3}$ is a benzenediyl group, and still more preferably a benzene-1 ,4-diyl group. The waved line marks the bond which attaches the group $R^S$ to $R^C$ in formula (1).

As shown in the above formula, the moiety $R^S$ comprises a functional group -C(O)- which is suitable to form an amide bond -C(O)-NH- or an alkylated amide bond -C(O)-NR-, preferably an amide bond, with a complementary group contained in $R^C$.

Thus, it is preferred that $R^S$ in formula (1) is a group of the following structure (S-2):

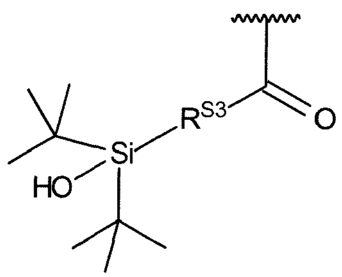

(S-2),

wherein

$R^{S3}$ is a group comprising a 6 membered aromatic ring, and is preferably benzenediyl, more preferably benzene-1,4-diyl; and

the waved line marks the bond which attaches the group $R^S$ to $R^C$ in formula (1).

In line with the above, and most preferably, $R^S$ in formula (1) is a moiety of the following structure (S-3):

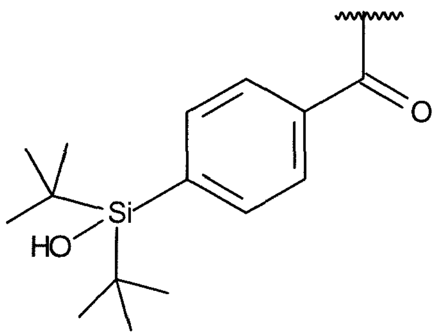

(S-3)

wherein

the waved line marks the bond which attaches the group $R^S$ to $R^C$ in of formula (1).

$R^A$ in formula (1) is an amino acid unit. As will be understood by the skilled person, an amino acid unit is a group which can be derived from an amino acid, i.e. from a compound comprising an amino group and a carboxylic acid group in the same molecule. Unless indicated otherwise in a specific context, one or more further functional groups in addition to the amino group and the carboxylic acid group may be present in the amino acid from which the amino acid unit can be derived. A specific amino acid unit is typically identified by the name of the amino acid from which it can be derived, e.g. as a glycine unit, asparagine unit, etc. Unless indicated otherwise in a specific context, the amino acid from which the amino acid unit $R^A$ can be derived is preferably an $\alpha$-amino acid. If an amino acid unit $R^A$ can be derived from a chiral amino acid, preference is given to the D-configuration.

The amino acid unit $R^A$ is typically derived from an amino acid by using an amino group of the amino acid to provide a functional group -NH- which forms an amide bond -C(O)-NH- or an alkylated amide bond -C(O)-NR-, preferably an amide bond, with a complementary group contained in one of the adjacent groups of formula (1), and by using a carboxylic acid group to provide a functional group -C(O)- which forms an amide bond -C(O)-NH- or an alkylated amide bond -C(O)-NR-, preferably an amide bond, with a complementary group contained in the other adjacent group of formula (1). Preferably, the amino acid unit $R^A$ provides a functional group -C(O)- to form an amide bond with a group -NH- provided by $R^C$, and a functional group -NH- to form an amide bond with a group -C(O)- provided by $R^{CH}$. More preferably, the functional group -NH- provided by the amino acid unit $R^A$ to form the amide bond with $R^{CH}$ is derived from an amino group in $\alpha$-position relative to the carboxylic acid group which provides the functional group -C(O)- to form the amide bond with $R^C$.

Preferably, the amino acid unit $R^A$ has the following structure (A-1):

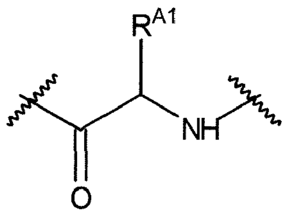

(A-1)

wherein

the waved line at the -C(O)- group shown in the formula marks a bond formed with $R^C$, the waved line at the -NH- group shown in the formula marks a bond formed with $R^{CH}$, and

$R^{A1}$ is selected from hydrogen, $-(CH_2)_k$-COOH, $-CH_2$-Ar, and -Ar, wherein k is 1, 2 or 3, preferably 2,

and Ar is an optionally substituted phenyl group, which may carry a substituent selected from -OH and $-NH_2$. If the phenyl group Ar is substituted, it is preferred that it carries a single substituent selected from -OH and $-NH_2$. It is preferred that the -C(O)- group shown in formula (A-1) forms an amide bond with a group -NH- provided by $R^C$, and that the -NH- group shown in formula (A-1) forms an amide bond with a group -C(O)- provided by $R^{CH}$.

More preferably, the amino acid unit $R^A$ has the following structure (A-1A):

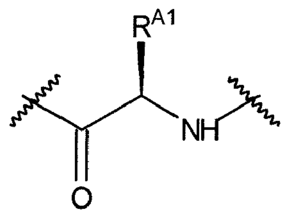

(A-1A)

wherein

the waved line at the -C(O)- group shown in the formula marks a bond formed with $R^C$, the waved line at the -NH- group shown in the formula marks a bond formed with $R^{CH}$, and $R^{A1}$ is defined as in formula (A-1). It is preferred that the -C(O)- group shown in formula (A-1A) forms an amide bond with a group -NH- provided by $R^C$, and that the -NH- group shown in formula (A-1A) forms an amide bond with a group -C(O)- provided by $R^{CH}$.

In the above formulae (A-1) and (A-1A), respectively, $R^{A1}$ is preferably selected from $-(CH_2)_2-COOH$, $-CH_2-Ph$, and $-CH_2-(p-NH_2-Ph)$, with Ph being a phenyl group. Most preferably, $R^{A1}$ is $-(CH_2)_2-COOH$.

As examples of suitable amino acid units $R^A$, reference can be made to a unit derived from Gly, Glu, Phe, (4-$NH_2$)-Phe, Tyr or Phenylglycin, with the chiral amino acids being preferably in D-configuration.

As will be understood by the skilled person, an amino acid unit comprising a side chain with a basic group such as $-NH_2$ or an acidic group such as -COOH may be protonated or deprotonated, respectively, depending on the conditions under which the compounds in accordance with the invention are kept, e.g. the pH of a solution thereof. A positive charge of a protonated amino group may be balanced, e.g., by a positive charge carried by another group in a compound in accordance with the invention, or by an anionic counterion, e.g., as discussed below with regard to salt forms of the compounds in accordance with the invention. A negative charge of a deprotonated carboxylic acid group may be balanced, e.g., by a positive charge carried by another group in a compound in accordance with the invention, or by a cationic counterion, e.g., as discussed below with regard to salt forms of the compounds in accordance with the invention.

$R^{CH}$ is selected from

(i) a branched-chain, acyclic chelating moiety having 4 amino groups, and

(ii) a chelate moiety, wherein a radioisotope selected from $^{99m}Tc$, $^{94m}Tc$, $^{186}Re$ and $^{188}Re$ is chelated by the branched-chain, acyclic chelating moiety having 4 amino groups.

Each of the 4 amino groups of the branched-chain, acyclic chelating moiety having 4 amino groups provides an electron lone pair suitable for forming a metal/ligand coordination bond, and the branched chain, acyclic chelating moiety is typically a tetradentate chelating moiety. As will be understood by the skilled reader, the chelating moiety is referred to as an acyclic chelating moiety since the coordinating nitrogen atoms of the 4 amino groups do not form ring members of a cyclic structure. As will further be understood, the reference to the chelating moiety as being a branched-chain chelating moiety requires the presence of at least one carbon atom in the chelating moiety which forms covalent bonds to at least three other carbon atoms. Unlike other chelating moieties used for providing Tc-chelates, such as the mas3 chelating moiety used in PSMA I&S, the branched-chain, acyclic chelating moiety having 4 amino groups defined above is typically free of a sulfur atom.

Preferably, the branched-chain, acyclic chelating moiety having 4 amino groups comprises a N4 chelating group of the following structure (CH-1):

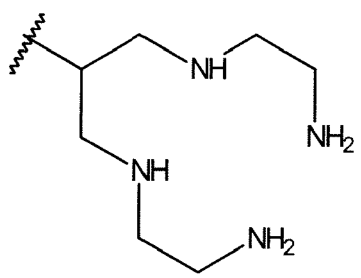

(CH-1),

wherein the waved line marks the bond which attaches the group to the remainder of the compound in accordance with the invention, and wherein optionally one or more, such as one, two or three hydrogen atoms attached to carbon atoms in the above formula can be replaced by a substituent, such as a methyl group. However, it is preferred that such

substituents are absent.

In addition to the amino groups allowing the formation of a chelate complex, the chelating moiety $R^{CH}$ generally comprises a functional group which is suitable to form an amide bond -C(O)-NH- or an alkylated amide bond -C(O)-NR-, preferably an amide bond, with a complementary group contained in $R^A$. Preferably this functional comprised by $R^{CH}$ is a group -C(O)-.

Thus, it is more preferred that the branched-chain, acyclic chelating moiety having 4 amino groups of $R^{CH}$ has the following structure (CH-2):

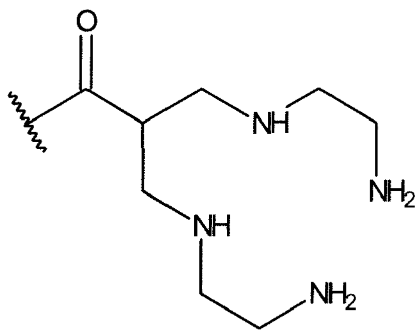

(CH-2)

wherein the waved line marks the bond which attaches the group to the remainder of the compound in accordance with the invention, and wherein optionally one or more, such as one, two or three hydrogen atoms attached to carbon atoms in the above formula can be replaced by a substituent, such as a methyl group. However, it is preferred that such substituents are absent.

In accordance with option (ii) above, the branched-chain, acyclic chelating moiety having 4 amino groups as discussed above may provide a chelate moiety wherein a radioisotope selected from $^{99m}$Tc, $^{94m}$Tc, $^{186}$Re and $^{188}$Re is chelated by the chelating moiety. Thus, the chelating moiety forms multiple (generally 4) coordinating bonds with the chelated radioisotope. In addition, one or more further ligands may be coordinated to the radioisotope. Compounds in accordance with the invention comprising a chelate moiety are also referred to herein as chelate compounds.

Preferably, the chelated radioisotope is $^{99m}$Tc.

In the chelate moiety and the chelate compounds comprising it, the chelated radioisotope is typically a cationic species, e.g. $^{99m}$Tc, $^{94m}$Tc, $^{186}$Re or $^{188}$Re in an oxidation state of +V. The chelated radioisotope may carry one or more, such as one, two or three additional ligands other than the chelating moiety which is contained in the compounds in accordance with the invention. For example, in a chelate moiety comprising a Tc cation (i.e. a $^{99m}$Tc cation or a $^{94m}$Tc cation, preferably in the oxidation state +V), a Tc cation complexed by the chelating moiety as a chelating ligand may carry e.g. two or more anionic ligands as additional ligands, such as two oxo ligands. Likewise, in a chelate moiety comprising a Re cation (i.e. a $^{186}$Re cation or a $^{188}$Re cation, preferably in the oxidation state +V), the Re cation complexed by the chelating moiety as a chelating ligand may carry e.g. two or more anionic ligands as additional ligands, such as two oxo ligands.

Thus, the $^{99m}$Tc, $^{94m}$Tc, $^{186}$Re or $^{188}$Re radioisotope is preferably chelated by the chelating moiety contained in the compounds in accordance with the invention as a dioxo-species of the formula ($[^{99m}Tc]TcO_2)^+$, ($[^{94m}Tc]TcO_2)^+$, ($[^{186}Re]ReO_2)^+$, or ($[^{186}Re]ReO_2)^+$, respectively.

A preferred chelate moiety in accordance with option (ii) above in the definition of $R^{CH}$ therefore comprises a group of the following structure (CH-3):

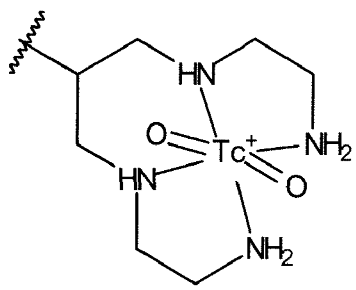

(CH-3)

wherein the waved line marks the bond which attaches the group to the remainder of the compound in accordance with

the invention, wherein the Tc is $^{99m}$Tc or $^{94m}$Tc and wherein optionally one or more, such as one, two or three hydrogen atoms attached to carbon atoms in the above formula can be replaced by a substituent, such as a methyl group. However, it is preferred that such substituents are absent.

In line with the above, it is more preferred that chelate moiety of $R^{CH}$ has the following structure (CH-4):

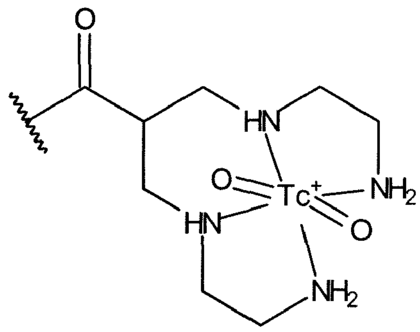

(CH-4)

wherein the waved line marks the bond which attaches the group to the remainder of the compound in accordance with the invention, wherein the Tc is $^{99m}$Tc or $^{94m}$Tc, and wherein optionally one or more, such as one, two or three hydrogen atoms attached to carbon atoms in the above formula can be replaced by a substituent, such as a methyl group. However, it is preferred that the substituents are absent.

If the chelate moiety is a charged moiety, e.g. a cationic moiety as illustrated in formulae (CH-3) and (CH-4), the positive charge may be balanced, e.g., by a negative charge carried by another group in a compound in accordance with the invention, or by an anionic counterion, e.g., as discussed below with regard to salt forms of the compounds in accordance with the invention. As an exemplary counterion, reference can be made to any anion which is used in sterile solutions for injection, such as $Cl^-$, $HPO_4^{2-}$, $PO_4^{3-}$, $H_2PO_4^-$, a citrate anion or an ascorbate anion.

Compounds in accordance with the invention which comprise a chelate moiety wherein a radioisotope selected from $^{99m}$Tc, $^{94m}$Tc, $^{186}$Re and $^{188}$Re is chelated may be referred to herein as radiolabeled compounds (or more specifically as $^{99m}$Tc-labeled, $^{94m}$Tc-labeled, $^{186}$Re-labeled or $^{188}$Re-labeled compounds, respectively). In contrast, compounds in accordance with the invention which comprise a chelating moiety without a chelated radioisotope may be referred to herein as unlabeled or non-labeled compounds.

As will be understood, the radiolabeled compounds in accordance with the invention are suitable as radiopharmaceuticals, e.g. in radiotherapy, and/or as radiotracers in diagnosis or radiosurgery. Non-labeled compounds in accordance with the invention provide, e.g., useful precursors for radiolabeling.

In line with the above explanations, the compound of formula (1) is preferably a compound of the following formula (1B):

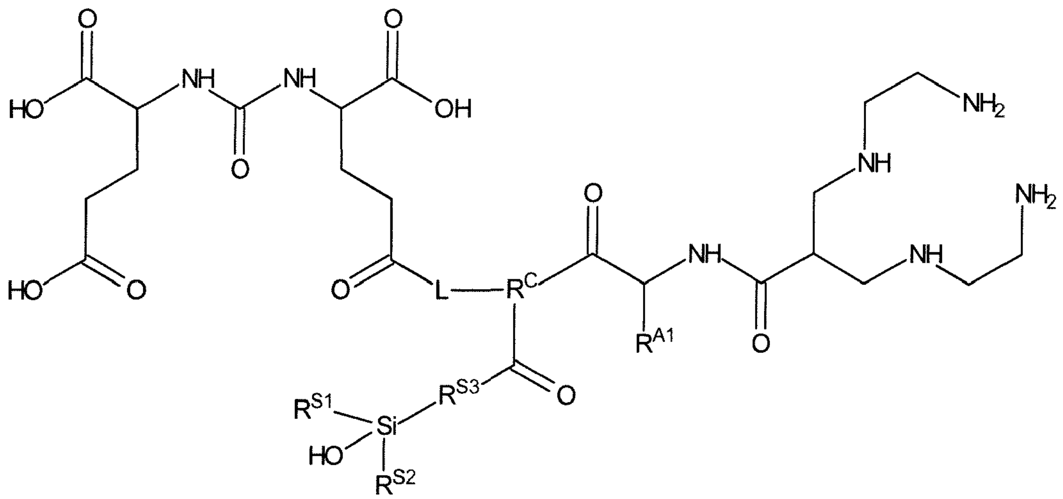

(1B),

wherein

the groups L, $R^C$, $R^{S1}$, $R^{S2}$, $R^{S3}$ and $R^{A1}$ are as defined hereinabove, including any preferred definitions thereof, and optionally one or more, such as one, two or three hydrogen atoms attached to carbon atoms in the chelating moiety $-C(O)-CH(CH_2NHCH_2CH_2NH_2)_2$ comprised in the above formula can be replaced by a substituent, such as a methyl group;

or a chelate compound wherein a radioisotope selected from $^{99m}Tc$, $^{94m}Tc$, $^{186}Re$ and $^{188}Re$ is chelated by the optionally substituted chelating moiety $-C(O)-CH(CH_2NHCH_2CH_2NH_2)_2$ comprised in the above formula (1B).

More preferably, the compound of formula (1) is a compound of formula (1BB):

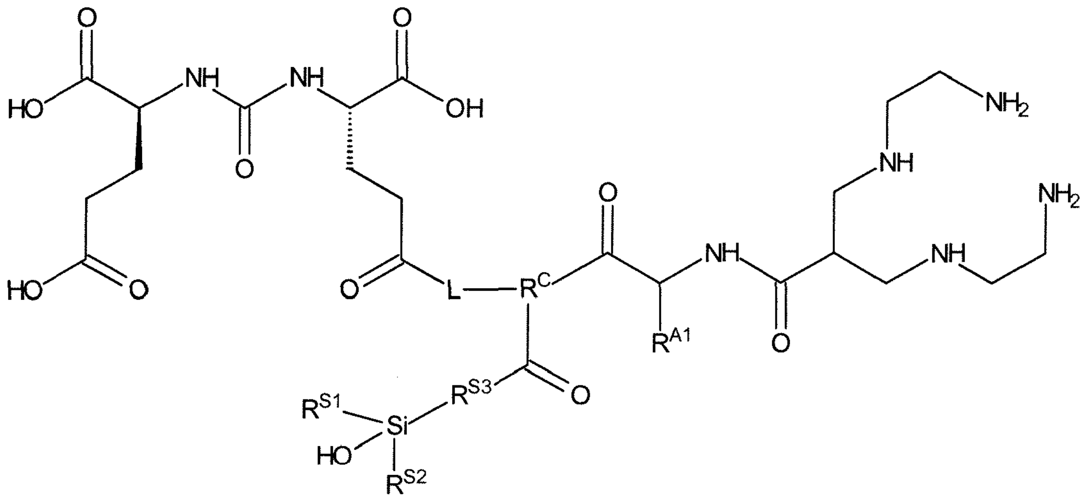

(1BB)

wherein

the groups L, $R^C$, $R^{S1}$, $R^{S2}$, $R^{S3}$ and $R^{A1}$ are as defined hereinabove, including any preferred definitions thereof, and optionally one or more, such as one, two or three hydrogen atoms attached to carbon atoms in the chelating moiety $-C(O)-CH(CH_2NHCH_2CH_2NH_2)_2$ comprised in the above formula can be replaced by a substituent, such as a methyl group;

or a chelate compound wherein a radioisotope selected from $^{99m}Tc$, $^{94m}Tc$, $^{186}Re$ and $^{188}Re$ is chelated by the optionally substituted chelating moiety $-C(O)-CH(CH_2NHCH_2CH_2NH_2)_2$ comprised in the above formula.

Even more preferably, the compound of formula (1) is a compound of formula (1C):

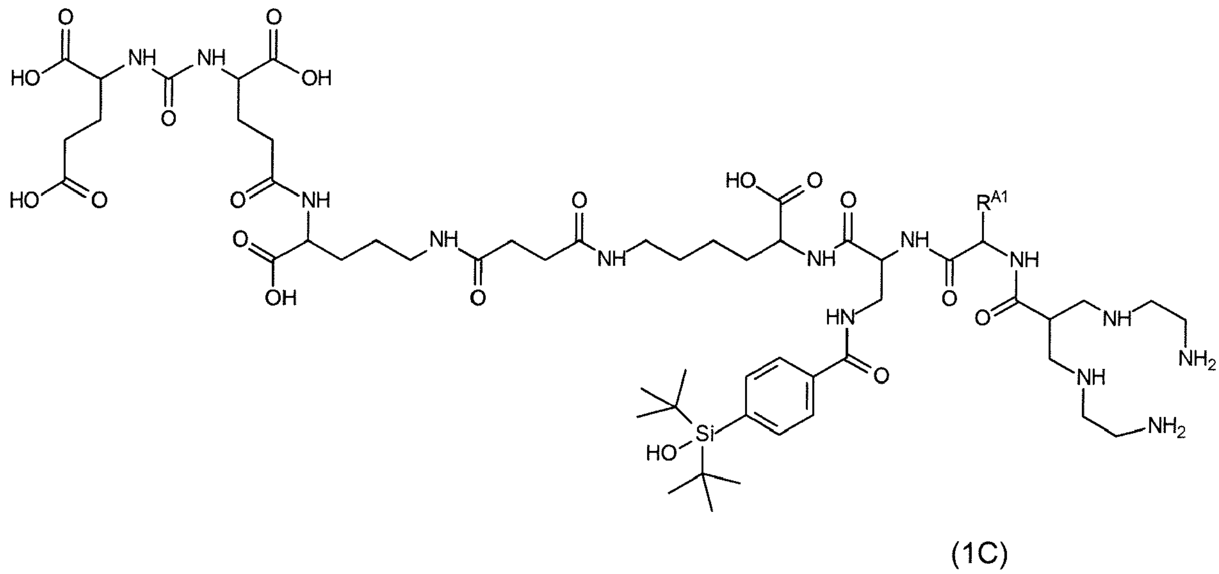

(1C)

wherein

the group $R^{A1}$ is as defined hereinabove, including any preferred definitions thereof, and optionally one or more, such as one, two or three hydrogen atoms attached to carbon atoms in the chelating moiety $-C(O)-CH(CH_2NHCH_2CH_2NH_2)_2$ comprised in the above formula can be replaced by a substituent, such as a methyl group;
or a chelate compound wherein a radioisotope selected from $^{99m}Tc$, $^{94m}Tc$, $^{186}Re$ and $^{188}Re$ is chelated by the optionally substituted chelating moiety $-C(O)-CH(CH_2NHCH_2CH_2NH_2)_2$ comprised in the above formula (1C).

Still more preferably, the compound of formula (1) is a compound of formula (1CC):

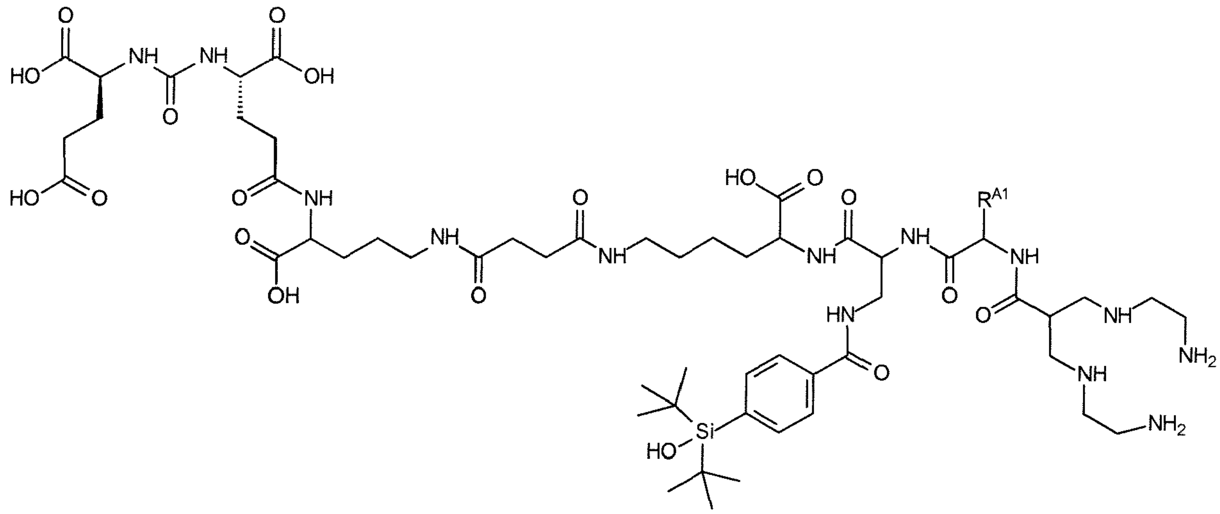

(1CC)

wherein

the group $R^{A1}$ is as defined hereinabove, including any preferred definitions thereof, and optionally one or more, such as one, two or three hydrogen atoms attached to carbon atoms in the chelating moiety $-C(O)-CH(CH_2NHCH_2CH_2NH_2)_2$ comprised in the above formula can be replaced by a substituent, such as a methyl group;
or a chelate compound wherein a radioisotope selected from $^{99m}Tc$, $^{94m}Tc$, $^{186}Re$ and $^{188}Re$ is chelated by the optionally substituted chelating moiety $-C(O)-CH(CH_2NHCH_2CH_2NH_2)_2$ comprised in the above formula.

As specific examples of compounds in accordance with the invention, reference can be made to the following compounds, to the corresponding chelate compounds wherein a radioisotope selected from $^{99m}Tc$, $^{94m}Tc$, $^{186}Re$ and $^{188}Re$ is chelated by the chelating moiety $-C(O)-CH(CH_2NHCH_2CH_2NH_2)_2$ comprised in the formula of these compounds, and to salts of the compounds or chelate compounds:

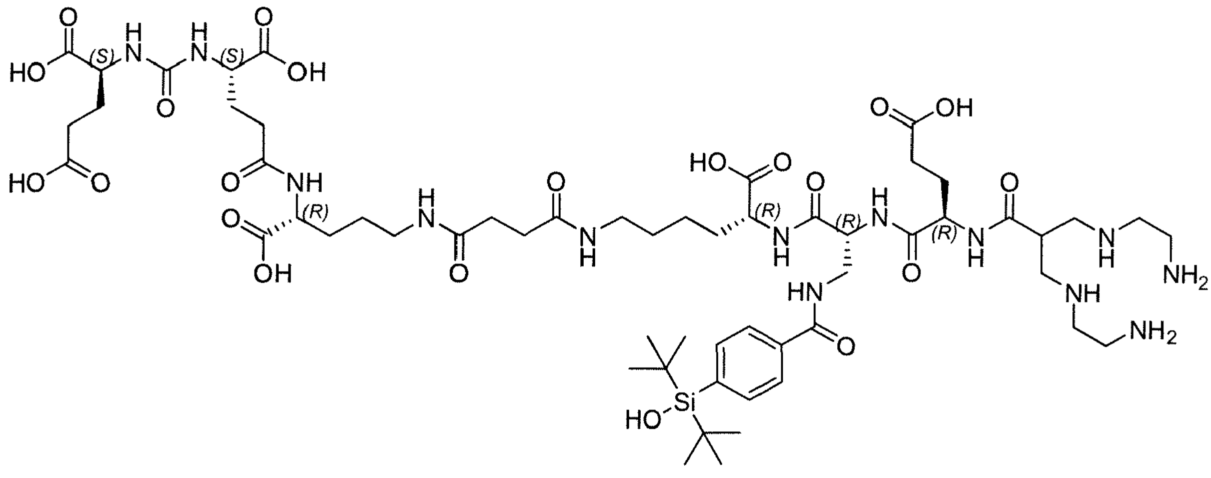

Chemical Formula: $C_{57}H_{95}N_{13}O_{21}Si$
Molecular Weight: 1326,54

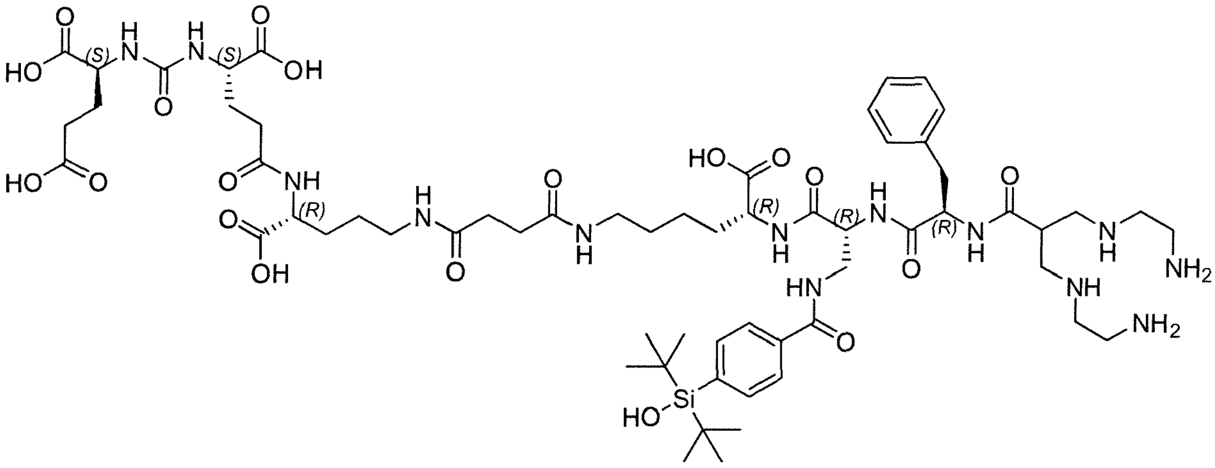

Chemical Formula: $C_{61}H_{97}N_{13}O_{19}Si$
Molecular Weight: 1344,60

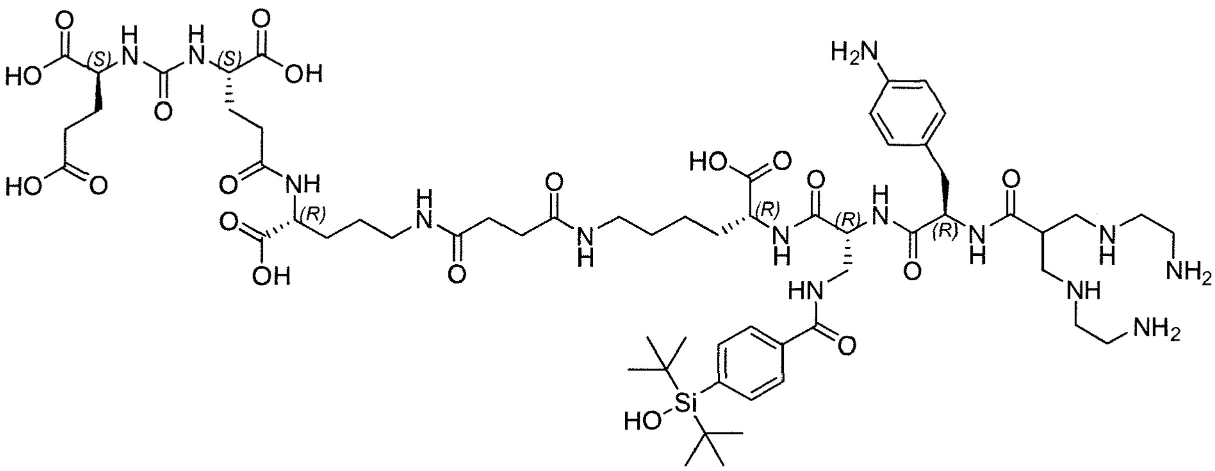

Chemical Formula: $C_{61}H_{98}N_{14}O_{19}Si$
Molecular Weight: 1359,62

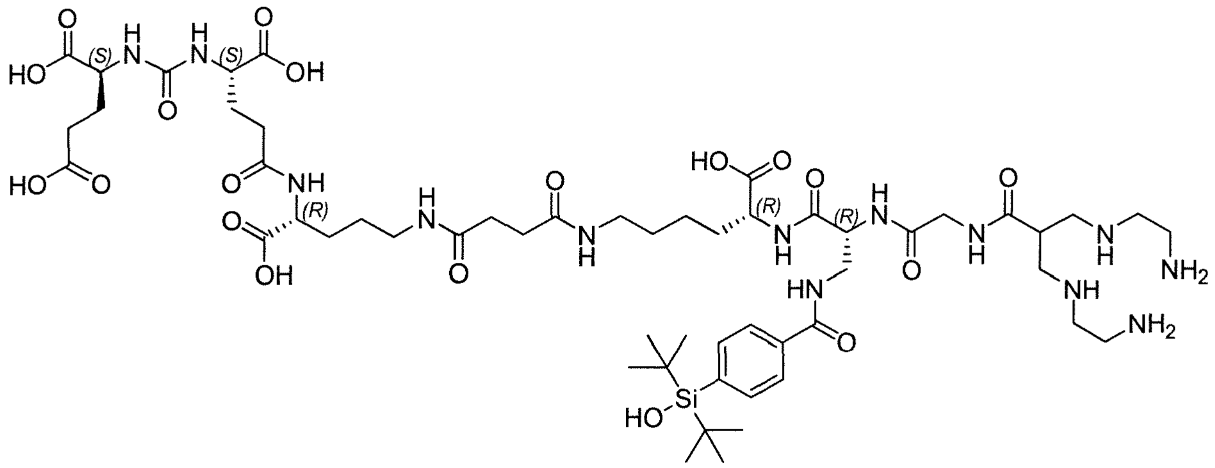

Chemical Formula: $C_{54}H_{91}N_{13}O_{19}Si$
Molecular Weight: 1254,48

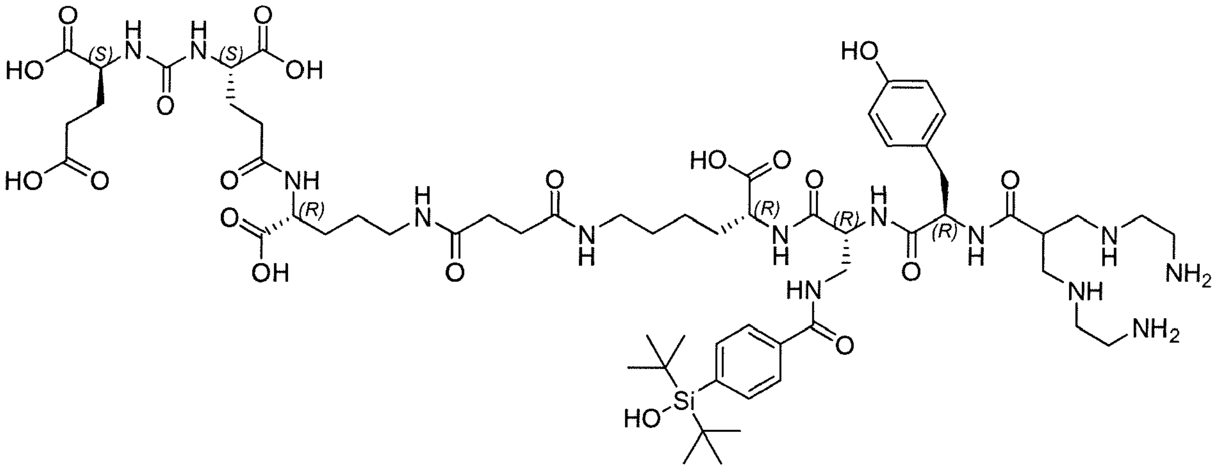

Chemical Formula: $C_{61}H_{97}N_{13}O_{20}Si$
Molecular Weight: 1360,60

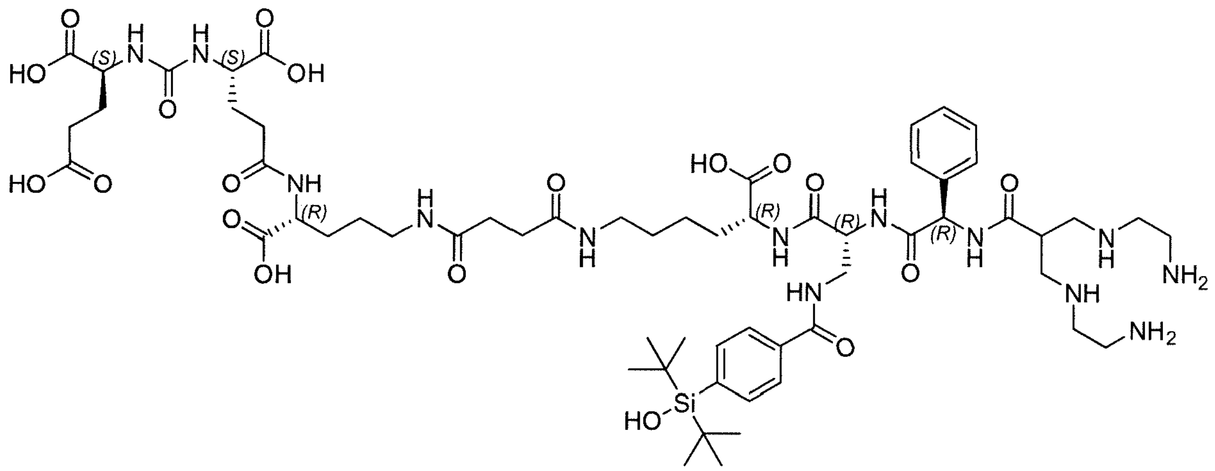

Chemical Formula: $C_{60}H_{95}N_{13}O_{19}Si$
Molecular Weight: 1330,58

As noted above, the compounds in accordance with the invention encompass the compounds of formula (1)

(including any preferred embodiments thereof, such as compounds of formula (1A), (1AA), (1B), (1BB), (1C), or (1CC)) and their salts. Salts are preferably pharmaceutically acceptable salts, i.e. formed with pharmaceutically acceptable anions or cations. Salts may be formed, e.g., by protonation of an atom carrying an electron lone pair which is susceptible to protonation, such as a nitrogen atom, with an inorganic or organic acid, or by separating a proton from an acidic group, such as a carboxylic acid group, e.g. by neutralization with a base. Other charged groups which may be present in the compounds in accordance with the invention and which may provide the compounds in the form of a salt include groups which are continuously charged, such as a charged chelate moiety.

As exemplary anions which may be present as counterions in salt forms of the compounds of the invention if the salt form comprises a positively charged form of the compound of formula (1), mention may be made, for example, of an anion selected from chloride, bromide, iodide, sulfate, nitrate, phosphate (such as, e.g., phosphate, hydrogenphosphate, or dihydrogenphosphate salts), carbonate, hydrogencarbonate or perchlorate; acetate, trifluoroacetate, propionate, butyrate, pentanoate, hexanoate, heptanoate, octanoate, cyclopentanepropionate, undecanoate, lactate, maleate, oxalate, fumarate, tartrate, malate, citrate, nicotinate, benzoate, salicylate or ascorbate; sulfonates such as methanesulfonate, ethanesulfonate, 2-hydroxyethanesulfonate, benzenesulfonate, p-toluenesulfonate (tosylate), 2-naphthalenesulfonate, 3-phenylsulfonate, or camphorsulfonate. Since trifluoroacetic acid is frequently used during the synthesis of peptides, trifluoroacetate salts are typical salts which are provided if a compound comprising an oligoamide structure is formed. Such trifluoroacetate salts may be converted e.g. to acetate salts during their workup.

As exemplary cations which may be present as counterions in salt forms of the compounds of the invention if the salt form comprises a negatively charged form of the compound of formula (1), mention may be made, for example, of a cation selected from alkali metal cations, such as lithium, sodium or potassium, alkaline earth metal cations, such as calcium or magnesium; and ammonium (including ammonium ions substituted by organic groups).

In addition to the compounds in accordance with the invention as discussed above, provided is a method for the preparation of a radiolabeled compound in accordance with the invention, which method comprises the step of contacting a non-radiolabeled compound in accordance with the invention as a precursor compound with a radioisotope selected from $^{99m}$Tc, $^{94m}$Tc, $^{186}$Re and $^{188}$Re or with a compound comprising such a radioisotope. Preferably, a compound comprising a radioisotope is used in the context of the method which is selected from a [$^{99m}$Tc]pertechnetate, a [$^{94m}$Tc]pertechnetate, a [$^{186}$Re]perrhenate and a [$^{188}$Re]perrhenate.

For example, the step of contacting a non-radiolabeled compound in accordance with the invention as a precursor compound with a radioisotope selected from $^{99m}$Tc, $^{94m}$Tc, $^{186}$Re and $^{188}$Re or with a compound comprising such a radioisotope can be carried out by dissolving the precursor compound and the compound comprising the radioisotope in a common solvent, preferably in an aqueous solution.

As explained above, a non-radiolabeled compound in accordance with the invention is a compound in accordance with the invention which does not comprise a chelated radioisotope. A radiolabeled compound in accordance with the invention (or more specifically a $^{99m}$Tc-labeled, $^{94m}$Tc-labeled, $^{186}$Re-labeled or $^{188}$Re-labeled compound, respectively) is a compound in accordance with the invention which comprises a chelated radioisotope selected from $^{99m}$Tc, $^{94m}$Tc, $^{186}$Re and $^{188}$Re. In line with the above, the chelated radioisotope comprised by the radiolabeled compound in accordance with the invention is contained in a chelate moiety wherein the radioisotope is chelated by the branched-chain, acyclic chelating moiety having 4 amino groups as defined above, including any of the preferred forms of the chelating moiety, such as the moiety $-C(O)-CH(CH_2NHCH_2CH_2NH_2)_2$.

As further explained above, the PSMA-targeted ligand compounds in accordance with the present invention are useful for applications as radiotracers, e.g. in the context of radiodiagnosis or radioguided surgery, or in radiotherapeutic applications. For example, a radiolabeled compound in accordance with the invention, preferably a $^{99m}$Tc-labeled or $^{94m}$Tc-labeled compound in accordance with the invention, can be effectively used e.g. as a radiotracer in the diagnosis of a disease which is associated with the overexpression of PSMA, or as a radiotracer (also referred to as radioactive targeted probe in this context) in radioguided surgery. By means of radioguided surgery assisted by a radiolabeled compound in accordance with the invention, it is possible to identify and remove diseased tissue which is associated with the overexpression of PSMA.

Thus, a further aspect of the invention is represented by a composition, e.g. a therapeutic or a diagnostic composition comprising a compound in accordance with the invention, preferably a radiolabeled compound in accordance with the invention. More specifically, it is preferred that the therapeutic composition comprises a $^{186}$Re-labeled or $^{188}$Re-labeled compound in accordance with the invention, and that the diagnostic composition comprises a $^{99m}$Tc-labeled or $^{94m}$Tc-labeled compound in accordance with the invention.

In a first related aspect, the invention provides a radiolabeled compound in accordance with the invention, preferably a $^{99m}$Tc-labeled or $^{94m}$Tc-labeled compound in accordance with the invention, or a diagnostic composition comprising such a compound, for use in diagnosing in *vivo* a disease which is associated with the overexpression of PSMA. The disease associated with the overexpression of PSMA is preferably cancer, and more preferably prostate cancer. Diagnosing preferably involves nuclear medicine tomography, more preferably single-photon emission computed tomography (SPECT).

In a further related aspect, the invention provides a radiolabeled compound in accordance with the invention, preferably a $^{99m}$Tc-labeled or $^{94m}$Tc-labeled compound in accordance with the invention, or a diagnostic composition comprising such a compound, for use in identifying *in vivo* diseased tissue which is associated with the overexpression of PSMA. In a preferred embodiment, the compound or the composition is provided for use in identifying *in vivo* diseased tissue which is associated with the overexpression of PSMA in the context of a radioguided surgery for removing the diseased tissue. The diseased tissue which is associated with the overexpression of PSMA is preferably cancer tissue, and more preferably prostate cancer tissue.

As still a further related aspect, the invention provides the *ex vivo* or *in vitro* use of a radiolabeled compound in accordance with the invention, preferably a $^{99m}$Tc-labeled or $^{94m}$Tc-labeled compound in accordance with the invention, or of a diagnostic composition comprising such a compound, for identifying a tissue or cell which is associated with the overexpression of PSMA, wherein the tissue or cell is preferably a cancer tissue or cell and more preferably a prostate cancer tissue or cell.

In accordance with another related aspect, an *ex vivo* or *in vitro* method is provided for identifying whether a tissue or cell overexpresses PSMA, said method comprising contacting the tissue or cell with a radiolabeled compound in accordance with the invention, preferably a $^{99m}$Tc-labeled or $^{94m}$Tc-labeled compound in accordance with the invention, or with a diagnostic composition comprising such a compound, wherein the tissue or cell is preferably a cancer tissue or cell and more preferably a prostate cancer tissue or cell.

In yet a further related aspect the invention provides a radiolabeled compound in accordance with the invention, preferably a $^{186}$Re-labeled or $^{188}$Re -labeled compound in accordance with the invention, or a therapeutic composition comprising such a compound, for use in treating or preventing a disease which is associated with the overexpression of PSMA, wherein the disease is preferably cancer and more preferably prostate cancer.

A composition comprising a compound in accordance with the invention, e.g. a therapeutic or diagnostic composition, may further comprise one or more pharmaceutically acceptable carriers, excipients and/or diluents. Examples of suitable pharmaceutical carriers, excipients and/or diluents are well known in the art and include phosphate buffered saline solutions, amino acid buffered solutions (with or without saline), water for injection, emulsions, such as oil/water emulsions, various types of wetting agents, sterile solutions etc. Compositions comprising such carriers can be formulated by well-known conventional methods. These compositions can be administered to the subject at a suitable dose. Administration of the suitable compositions may be accomplished in different ways, e.g., by intravenous injection and/or delivery. The compositions may be administered directly to the target site.

The following items summarize aspects of the invention. It will be understood that these items are closely related to the above parts of the description, and that the information provided in these items may supplement the above parts of the description and vice versa.

1. A compound of formula (1) or a salt thereof:

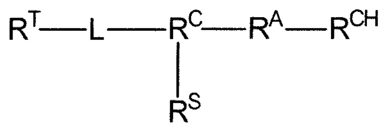

(1)

wherein

| | |
|---|---|
| $R^T$ | is a PSMA binding group; |
| L | is a linking group; |
| $R^C$ | is a trivalent coupling group; |
| $R^S$ | is a silicon-containing moiety of the formula -C(O)-$R^{S3}$-Si$R^{S1}R^{S2}$OH, wherein $R^{S1}$ and $R^{S2}$ are independently selected from C3-C10 alkyl, and $R^{S3}$ is a group comprising a 6 membered aromatic ring; |
| $R^A$ | is an amino acid residue; and |
| $R^{CH}$ | is selected from |

(i) a branched-chain, acyclic chelating moiety having 4 amino groups, and
(ii) a chelate moiety, wherein a radioisotope selected from $^{99m}$Tc, $^{94m}$Tc, $^{186}$Re and $^{188}$Re is chelated by the branched-chain, acyclic chelating moiety having 4 amino groups.

2. The compound or salt in accordance with item 1, wherein $R^T$ in formula (1) is a group of the following structure (T-1):

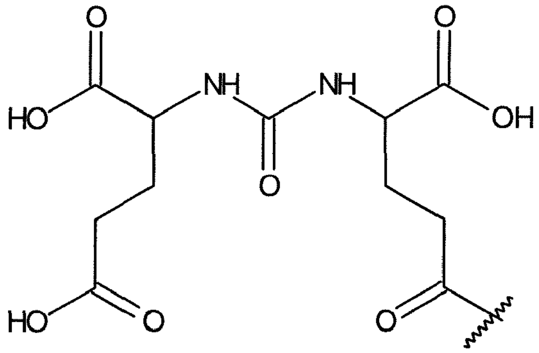

(T-1)

wherein the waved line marks the bond which attaches the group $R^T$ to the remainder of the compound of formula (1).

2. The compound or salt in accordance with item 2, wherein $R^T$ in formula (1) is a group of the following structure (T-2):

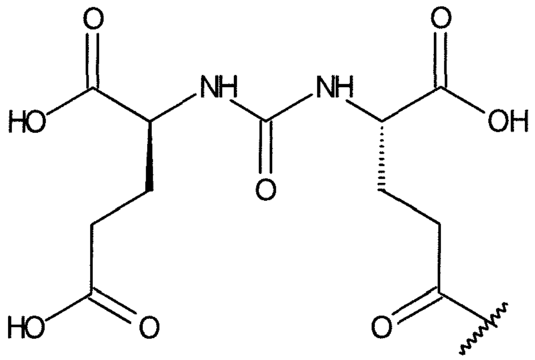

(T-2)

wherein the waved line marks the bond which attaches the group $R^T$ to the remainder of the compound of formula (1).

3. The compound or salt in accordance with item 1 or 2, wherein $R^S$ in formula (1) is a group of the following structure (S-2):

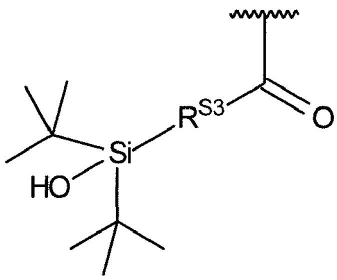

(S-2)

wherein

$R^{S3}$ is a group comprising a 6 membered aromatic ring; and
the waved line marks the bond which attaches the group $R^S$ to the remainder of the compound of formula (1).

4. The compound or salt in accordance with any of items 1 to 3, wherein $R^S$ in formula (1) is a group of the following structure (S-3):

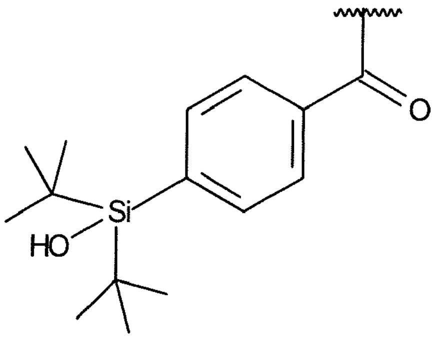

(S-3)

wherein
the waved line marks the bond which attaches the group $R^S$ to the remainder of the compound of formula (1).

5. The compound or salt in accordance with any of items 1 to 4, wherein $R^A$ in formula (1) is a group of the following structure (A-1):

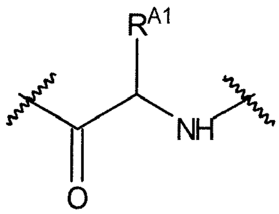

(A-1)

wherein

$R^{A1}$ is selected from hydrogen, $-(CH_2)_k-COOH$, $-CH_2-Ar$, and -Ar,
wherein k is 1, 2 or 3, preferably 2, and
Ar is an optionally substituted phenyl group, which may carry a substituent selected from -OH and $-NH_2$,
the waved line at the -C(O)- group shown in the formula marks a bond formed with $R^C$, and the waved line at the -NH- group shown in the formula marks a bond formed with $R^{CH}$.

6. The compound or salt in accordance with item 5, wherein $R^A$ in formula (1) is a group of the following structure (A-1A):

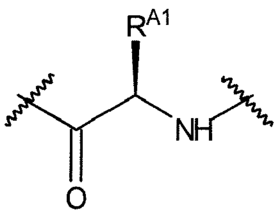

(A-1A)

wherein

$R^{A1}$ is selected from hydrogen, $-(CH_2)_k-COOH$, $-CH_2-Ar$, and -Ar,
wherein k is 1, 2 or 3, preferably 2, and
Ar is an optionally substituted phenyl group, which may carry a substituent selected from -OH and $-NH_2$,
the waved line at the -C(O)- group shown in the formula marks a bond formed with $R^C$, and the waved line at the -NH- group shown in the formula marks a bond formed with $R^{CH}$.

7. The compound or salt in accordance with item 5 or 6, wherein $R^{A1}$ is selected from $-(CH_2)_2-COOH$, $-CH_2-Ph$, and $-CH_2-(p-NH_2-Ph)$, with Ph being a phenyl group.

8. The compound or salt in accordance with item 7, wherein $R^{A1}$ is $-(CH_2)_2$-COOH.

9. The compound or salt in accordance with any of items 1 to 8, wherein $R^{CH}$ in formula (1) is selected from:

(i) a N4 chelating group of the formula:

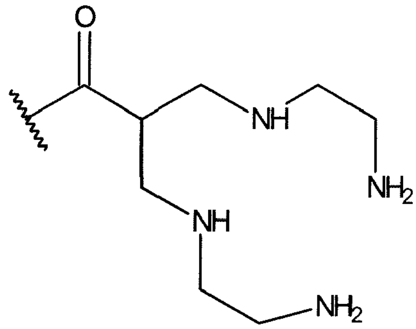

wherein
the waved line marks the bond which attaches the group $R^{CH}$ to the remainder of the compound of formula (1), and wherein optionally one or more, such as one, two or three hydrogen atoms attached to carbon atoms in the above formula can be replaced by a substituent, and
(ii) a chelate moiety, wherein a radioisotope selected from $^{99m}$Tc, $^{94m}$Tc, $^{186}$Re and $^{188}$Re is chelated by the N4 chelating group.

10. The compound or salt in accordance with any of items 1 to 9, wherein L in formula (1) is an oligoamide residue comprising 2 to 6 subunits linked to each other via an amide bond -C(O)-NH- or via an alkylated amide bond -C(O)-NR- wherein R is a C1-C6 alkyl group.

11. The compound or salt in accordance with item 10, wherein the oligoamide residue comprises 2 to 4 subunits.

12. The compound or salt in accordance with item 10 or 11, wherein the oligoamide residue comprises 6 to 20, preferably 8 to 18, and more preferably 8 to 16 carbon atoms, not including carbon atoms in optional substituent groups attached to the backbone.

13. The compound or salt in accordance with any of items 1 to 12, wherein $R^C$ in formula (1) is a trivalent amino acid unit which is derived from an amino acid comprising, together with the carboxylic acid group and the amino group, a side chain carrying a further functional group selected form a carboxylic acid group and an amino group.

14. The compound or salt in accordance with any of items 1 to 13, wherein $R^C$ in formula (1) is selected from a 2,3-diaminopropionic acid (Dap) unit, a 2,4-diaminobutanoic acid (Dab) unit, an ornithine (Orn) unit and a lysine (Lys) unit.

15. The compound or salt in accordance with any of items 1 to 14, which is a radiolabeled compound comprising a chelated radioisotope selected from $^{99m}$Tc, $^{94m}$Tc, $^{186}$Re and $^{188}$Re.

16. The compound or salt in accordance with item 15, wherein the chelated radioisotope is in the oxidation state +V.

17. The compound or salt in accordance with item 15 or 16, wherein the chelated radioisotope is chelated as a dioxo-species.

18. The compound or salt in accordance with any of items 15 to 17, wherein the chelated radioisotope is selected from $^{99m}$Tc and $^{94m}$Tc.

19. The compound or salt in accordance with any of items 15 to 17, wherein the chelated radioisotope selected from $^{186}$Re and $^{188}$Re.

20. A composition comprising a compound or salt in accordance with any of items 1 to 19.

21. The composition in accordance with item 20, which is a diagnostic composition.

22. The diagnostic composition in accordance with item 21, which comprises a compound or salt in accordance with item 18.

23. The compound or salt in accordance with any of items 1 to 19, preferably of item 18, or the diagnostic composition in accordance with item 21 or 22 for use in diagnosing *in vivo* a disease which is associated with the overexpression of PSMA.

24. The compound or salt or diagnostic composition for use in accordance with item 23, wherein the disease is cancer and preferably prostate cancer.

25. The compound or salt or diagnostic composition for use in accordance with item 23 or 24, wherein diagnosing involves nuclear medicine tomography, preferably single-photon emission computed tomography (SPECT).

26. The compound or salt in accordance with any of items 1 to 19, preferably of item 18, or the diagnostic composition in accordance with item 21 or 22 for use in identifying *in vivo* diseased tissue which is associated with the overexpression of PSMA.

27. The compound or salt or diagnostic composition for use in accordance with item 26, wherein the diseased tissue is cancer tissue and preferably prostate cancer tissue.

28. The compound or salt or the diagnostic composition for use in accordance with item 26 or 27, wherein the diseased tissue is identified in the context of a radioguided surgery for removing the diseased tissue.

29. *Ex vivo* or *in vitro* use of the compound or salt in accordance with any of items 1 to 19, preferably of item 18, or the diagnostic composition in accordance with item 21 or 22 for identifying a tissue or cell which is associated with the overexpression of PSMA.

30. The use in accordance with item 29, wherein the tissue or cell is a cancer tissue or cell and preferably a prostate cancer tissue or cell.

31. An *ex vivo* or *in vitro* method for identifying whether a tissue or cell overexpresses PSMA, comprising contacting the tissue or cell with the compound or salt in accordance with any of items 1 to 19, preferably of item 18, or the diagnostic composition in accordance with item 21 or 22.

32. The method in accordance with item 31, wherein the tissue or cell is a cancer tissue or cell and preferably a prostate cancer tissue or cell.

33. The composition in accordance with item 20, which is a therapeutic composition.

34. The therapeutic composition in accordance with item 33, which comprises a compound or salt in accordance with item 19.

35. The compound or salt in accordance with any of items 1 to 19, preferably of item 19, or the therapeutic composition in accordance with item 33 or 34 for use in treating or preventing a disease which is associated with the overexpression of PSMA.

36. The compound or salt or the therapeutic composition in accordance with item 35, wherein the disease is cancer and preferably prostate cancer.

In this specification, a number of documents including patent applications and manufacturer's manuals are cited. The disclosure of these documents, while not considered relevant for the patentability of this invention, is herewith incorporated by reference in its entirety. More specifically, all referenced documents are incorporated by reference to the same extent as if each individual document was specifically and individually indicated to be incorporated by reference.
The following examples further illustrate the invention without limiting it to the illustrated embodiments.

## Examples

## Materials and Methods

### *General Information*

Protected amino acids for peptide synthesis were purchased from Carbolution (St. Ingbert, Germany) and Iris Biotech (Marktredwitz, Germany). The 2-Chlorotrityl chloride polystyrene (TCP) resin was obtained from Sigma-Aldrich (Steinheim, Germany). Solvents and all other organic and inorganic reagents were purchased from Alfa Aesar (Karlsruhe, Germany), Fluorochem (Hadfield, United Kingdom), Sigma-Aldrich (Steinheim, Germany) or VWR (Darmstadt, Germany) and used without further purification. Radioactive [$^{99m}$Tc]$TcO_4^-$ was obtained from a Ultra-Technekow FM 2 (15 - 43.00 GBq) generator (Curium, Petten, Netherlands).

Solid phase peptide synthesis (SPPS) was carried out manually in syringe reactors for peptide synthesis (Carl Roth, Karlsruhe, Germany) using a MX-RD-Pro syringe shaker from SCILOGEX (Rocky Hill, United States). Analytical and preparative reversed-phase high-performance liquid chromatography (RP-HPLC) was performed using Shimadzu gradient systems (Shimadzu, Neufahrn, Germany) each equipped with a SPD-20A UV/Vis-detector (detection at $\lambda$ = 220 nm) and LC-20AD solvent pumps. Eluents for all chromatographic procedures were water (solvent A, 0.1% TFA *(v/v))* and acetonitrile (solvent B, 0.1% TFA (*v*/*v*), 2% or 5% water (*v*/*v*) in analytical or preparative procedures, respectively). For analytical measurements a MultoKrom 100-5 C18 column (150 mm $\times$ 4.6 mm, CS Chromatographie-Service, Langerwehe, Germany) was used at a constant flow rate of 1 mL/min. Preparative RP-HPLC was performed on a MultoKrom 100-5 C18 column (250 mm $\times$ 20 mm, CS Chromatographie-Service) applying a constant flow rate of 10 mL/min. Reversed-phase high performance flash chromatography (RP-HPFC) was performed on an SP HPFC system with SNAP cartridges (KP-C18-HS, 12 g) from Biotage (Charlottesville, United States) applying water (solvent A, 0.1% TFA *(v/v))* and acetonitrile (solvent B, 0.1% TFA *(v/v))* as eluents. Electrospray ionization (ESI) mass spectra and atmospheric pressure chemical ionization (APCI) mass spectra for compound characterization were acquired on an expression$^L$ CMS mass spectrometer from Advion (Harlow, United Kingdom). $^1$H-NMR-spectra were acquired on an AVHD 400 from Bruker (Billerica, United States) at 300 K. Chemical shifts ($\delta$) are given in parts per million (ppm), spectra are calibrated to the residual $^1$H solvent signal of DMSO-$d_6$ at 2.50 ppm and signal multiplicities are described as: s = singlet, m = multiplet.

Analytical and preparative radio RP-HPLC was performed on a Shimadzu system equivalent as stated above and additionally equipped with a SIL-20A HAT autosampler using a MultoKrom 100-5 C18 column (125 mm $\times$ 4.6 mm) from CS Chromatographie-Service at a constant flow rate of 1 mL/min. A HERM LB 500 NaI scintillation detector (Berthold Technologies, Bad Wildbad, Germany) was connected to the outlet of the UV-photometer for the detection of radioactivity. Radio thin layer chromatography (TLC) was performed on iTLC-SG stripes (Agilent Technologies, Waldbronn, Germany) using butanone or $NH_4OAc$ (1 M in water) with DMF (1/1 *(v/v))* as mobile phase for quantification of free [$^{99m}$Tc]$TcO_4^-$ or colloidal technetium-99m, respectively. Radio-TLC stripes were analyzed using a Scan-RAM Radio-TLC detector from LabLogic Systems (Sheffield, United Kingdom). Activity quantification of radioactive probes was carried out using a 2480 WIZARD$^2$ automatic gamma counter (PerkinElmer, Waltham, United States).

Centrifuges used for the determination of lipophilicity and binding to human plasma were a HERAEUS Pico 17 and a HERAEUS Megafuge 16R, respectively (Thermo Scientific, Osterode, Germany).

### *Solution state synthesis of building blocks for SPPS*

*(tBu)$_2$EuE(tBu):*

The tert-butyl protected Glu-urea-Glu binding motive was synthesized in analogy to the synthesis of tert-butyl protected Lys-urea-Glu reported in literature [28, 35].

*4-(Di-tert-butylhydroxysilyl)benzoic acid (SiOH-BA):*

4-(Di-tert-butylhydroxysilyl)benzoic acid (SiOH-BA) was obtained by hydrolysis of 4-(Di-tert-butylfluorosilyl)benzoic acid (SiFA-BA). The latter was synthesized according to a published procedure [36]. To a stirring solution of SiFA-BA (114 mg, 403 $\mu$mol, 1.0 eq.) in DMF (4 mL) a solution of KOH (113 mg, 2013 $\mu$mol, 5.0 eq.) in TP-water (1 mL) was added at room temperature and stirred for 1 h. The solution was acidified (pH 4-5) by addition of 2.013 mL 1 M $HCl_{(aq)}$ and extracted with diethyl ether (5x 5 mL). The combined organic phases were dried over $MgSO_4$ and solvents were evaporated *in vacuo*. Residual DMF was removed via lyophilization to obtain the product as colorless, amorphous solid (96%).

RP-HPLC (50-100% B in 15 min): $t_R$ = 5.8 min, K' = 2.90. Calculated monoisotopic mass ($C_{15}H_{24}O_3Si$): 280.2;

found: m/z (APCI) = 279.0 $[M-H]^-$.

*N,N',N",N‴-Tetrakis(tert-butyloxycarbonyl)-6-carboxy-1,4,8,11-tetraazaundecane ($(tBu)_4N_4$):* N-Boc-ethylenediamine (4.0 eq.) was slowly added to a solution of 3-bromo-2-(bromomethyl)-propionic acid (1.0 eq.) in THF (25 mL/mmol) and stirred for 24 h at room temperature. The solvent was removed *in vacuo* and the crude residue dissolved in acetone/$H_2O$ (1/1 (*v*/*v*), 25 mL/mmol). The solution was cooled to 0°C and triethylamine (3.0 eq.) was added. After 5 min di-tert-butyl dicarbonate (4.0 eq.) was added and the stirring mixture was left to warm up to room temperature within 15 h. Solvents were removed *in vacuo,* the raw product was purified via RP-HPFC (35-71% B in 15min) and the desired product was obtained as colorless, amorphous solid.

ESI-MS: calculated monoisotopic mass ($C_{28}H_{52}N_4O_{10}$): 604.4; found: m/z (ESI) = 605.5 $[M+H]^+$. $^1$H-NMR (400 MHz, DMSO-$d_6$) $\delta$ = 7.17-6.19 (m, 2H, NH), 3.28-3.17 (m, 6H, $CH_2$), 3.10-2.95 (m, 6H, $CH_2$), 2.94-2.90 (m, 1H, CH), 1.38 (s, 18H, $CH_3$), 1.36 (s, 18H, $CH_3$).

**Synthesis of PSMA ligands**

Chemical synthesis of novel N4-bearing PSMA-ligands was carried out via Fmoc-based standard solid phase peptide synthesis (SPPS). Reference compound PSMA-I&S was synthesized as described earlier [15]. Purity of all labeling precursors was determined via *RP*-HPLC (UV-detection at 220 nm) and was >98% in all cases.

*General procedures (GP) for solid phase peptide synthesis:*

All synthetic steps were carried out in a syringe reactor for peptide synthesis at room temperature. After each coupling or deprotection step, the resin was thoroughly washed with DMF (5 mL/g resin) six or eight times, respectively.

*TCP-resin loading (GP1):* The amino acid (2.0 eq.) and DIPEA (3.75 eq.) are dissolved in DMF (5 mL/g resin) and added to the TCP resin. After 2.5 h, methanol (2 mL/g resin) is added for capping of remaining trityl chloride groups. Subsequently, the resin is washed thoroughly with DMF (6x 5 mL/g resin), DCM (3x 5 mL/g resin) and methanol (3x 5 mUg resin) and dried *in vacuo.* The loading *l* of the amino acid is determined by the equation

$$l\left[\frac{mmol}{g}\right] = \frac{(m_2 - m_1)\cdot 1000}{(M_{AA} - M_{HCl})\cdot m_2}$$

with $m_1$ = mass of unloaded resin [g], $m_2$ = mass of loaded resin [g], $M_{AA}$ = molecular weight of amino acid [g/mol] and $M_{HCl}$ = molecular weight of HCl [g/mol].

*On-resin amide bond formation (GP2):* For the conjugation of Fmoc-protected amino acids and other building blocks, their carboxylic acid functionality is preactivated by addition of TBTU (2.0 eq.), HOAt (2.0 eq.) and DIPEA (6.0 eq.) in DMF. After 5 min the solution is added to the resin and left to react for 2.5 h (differing coupling times are mentioned in the synthesis protocol). Coupling of Fmoc-D-Dap(Dde)-OH is performed with 2,4,6-trimethylpyridine (6.7 eq.) as base instead of DIPEA to prevent racemization.

*On-resin Fmoc-deprotection (GP3):* Deprotection of Fmoc-protecting groups is achieved by addition of 20% piperidine in DMF (8 mL/g resin) for 5 min and subsequently for 15 min.

*N4-PSMA ligands:*

Fmoc-D-Orn(Dde)-OH was loaded to the TCP resin as first building block according to GP1, and after subsequent Fmoc cleavage (GP3), (*t*BuO)EuE(O*t*Bu)$_2$ was conjugated for 4.5 h (GP2). Deprotection of Dde was carried out using a solution of 2% hydrazine monohydrate in DMF (5 mL/g resin) for 20 min. Subsequently, a solution of succinic anhydride (7 eq.) and DIPEA (7 eq.) in DMF was added and left to react for 2.5 h. The resin bound carboxylate was then preactivated by addition of TBTU (2.0 eq.), HOAt (2.0 eq.) and DIPEA (6.0 eq.) in DMF for 30 min and Fmoc-D-Lys-OtBu (2.0 eq.) in DMF was added for conjugation for 2.5 h. Subsequent Fmoc deprotection (GP3) was followed by conjugation of Fmoc-D-Dap(Dde)-OH (GP2). Orthogonal deprotection of Dde was carried out using hydroxylamine hydrochloride (1.26 g/g resin) and imidazole (0.92 g/g resin) in a mixture of DMF (1 mL/g resin) and NMP (5 mL/g resin) for 3.5 h. Subsequently, SiOH-BA was conjugated (GP2) and the remaining Fmoc protecting group was cleaved according to GP3. In the following step, either Fmoc-D-Glu(OtBu)-OH (in N4-PSMA-12), Fmoc-D-Phe-OH (in N4-PSMA-13) or Fmoc-D-Phe(4-NHBoc)-OH (in N4-PSMA-21) was coupled according to GP2. After subsequent Fmoc-deprotection (GP3), the tert-butyl protected N4-chelator was conjugated according to GP2. Cleavage from the resin and simultaneous deprotection of the ligand was performed in TFA (+2.5% TIPS, +2.5% $H_2O$) for 1 h. After purification by semipreparative *RP*-HPLC, N4-PSMA-12, N4-PSMA-13 and N4-PSMA-21 were obtained as colorless, amorphous solids in yields of 29%, 25% and 21%, respectively (yields refer to the amount of substance of resin-bound Fmoc-D-Orn(Dde) at the start of solid phase synthesis).

N4-PSMA-12: RP-HPLC (10-60% B in 15 min): $t_R$ = 9.3 min, K' = 3.72. Calculated monoisotopic mass ($C_{57}H_{95}N_{13}O_{21}Si$): 1325.7; found: m/z (ESI) = 1326.5 $[M+H]^+$, 664.0 $[M+2H]^{2+}$.

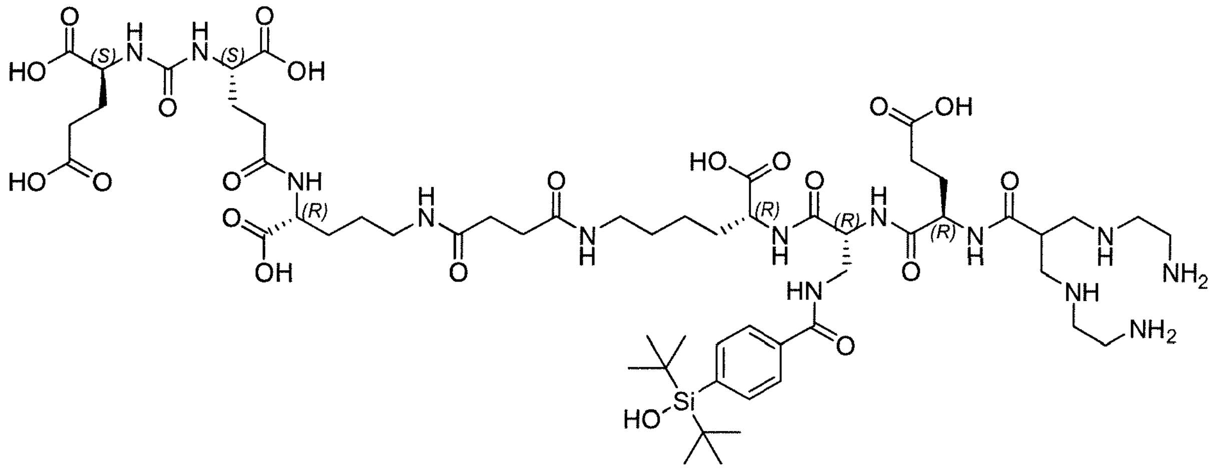

Chemical Formula: $C_{57}H_{95}N_{13}O_{21}Si$
Molecular Weight: 1326,54

N4-PSMA-13: RP-HPLC (10-60% B in 15 min): $t_R$ = 10.2 min, K' = 4.08. Calculated monoisotopic mass ($C_{61}H_{97}N_{13}O_{19}Si$): 1343.7; found: m/z (ESI) = 1344.4 $[M+H]^+$, 673.0 $[M+2H]^{2+}$.

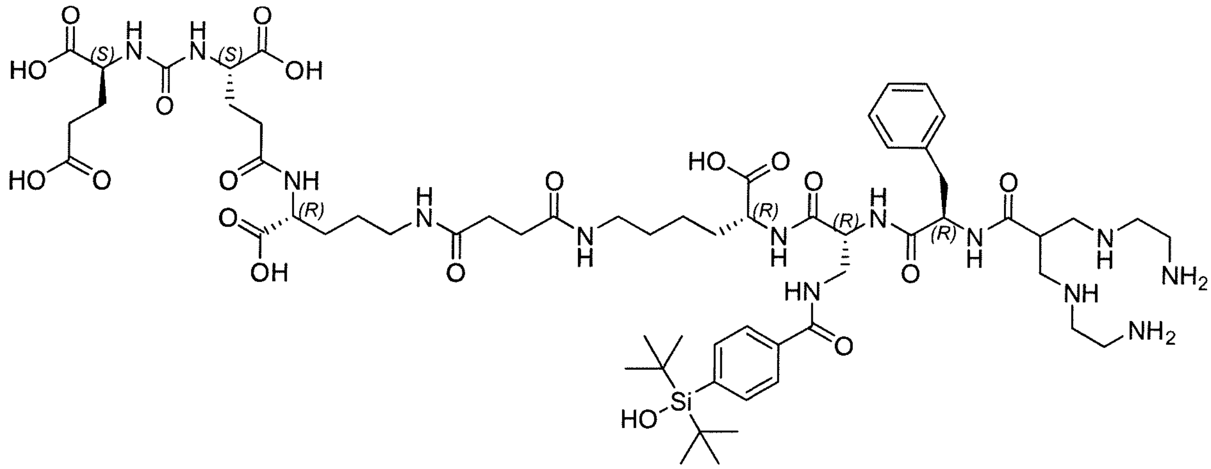

Chemical Formula: $C_{61}H_{97}N_{13}O_{19}Si$
Molecular Weight: 1344,60

N4-PSMA-21: RP-HPLC (10-60% B in 15 min): $t_R$ = 9.0 min, K' = 3.60. Calculated monoisotopic mass ($C_{61}H_{98}N_{14}O_{19}Si$): 1358.7; found: m/z (ESI) = 1359.7 $[M+H]^+$, 680.5 $[M+2H]^{2+}$.

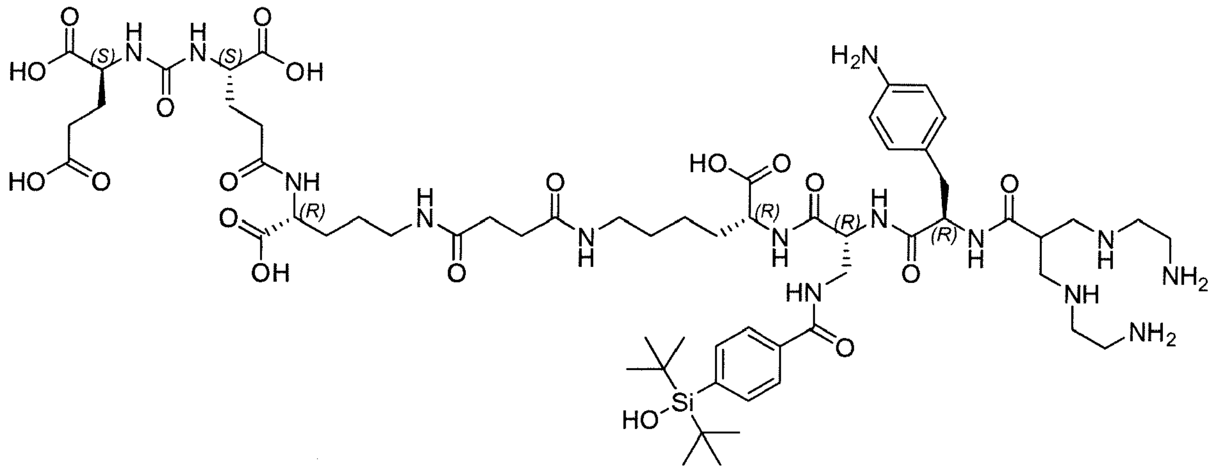

Chemical Formula: $C_{61}H_{98}N_{14}O_{19}Si$
Molecular Weight: 1359,62

The following ligands were prepared according to corresponding procedures.

N4-PSMA-11:

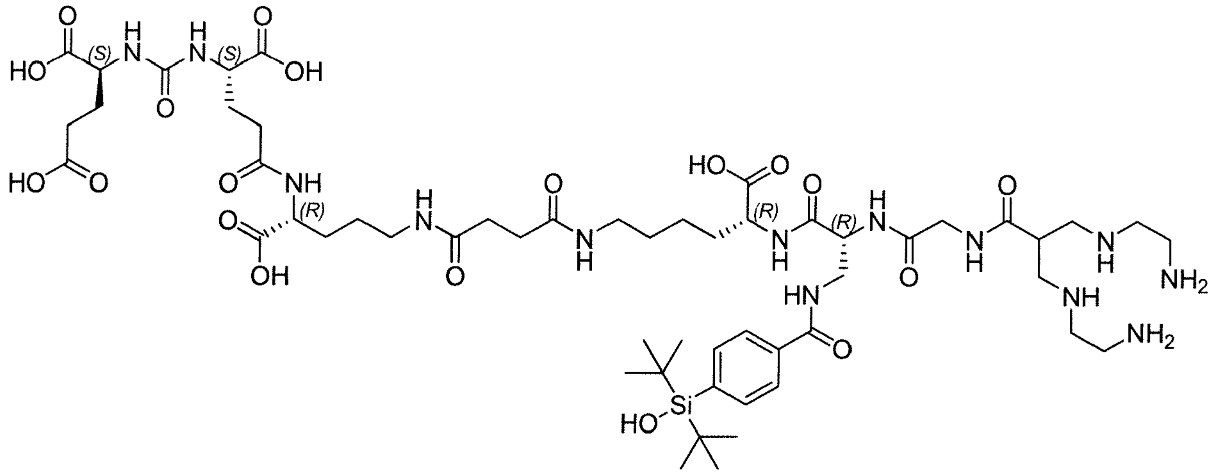

Chemical Formula: $C_{54}H_{91}N_{13}O_{19}Si$
Molecular Weight: 1254,48

N4-PSMA-11: RP-HPLC (10-60% B in 15 min): $t_R$ = 8.8 min, K' = 3.52. Calculated monoisotopic mass ($C_{54}H_{91}N_{13}O_{19}Si$): 1253.6; found: m/z (ESI) = 1255.0 $[M+H]^+$, 627.8 $[M+2H]^{2+}$.

N4-PSMA-32:

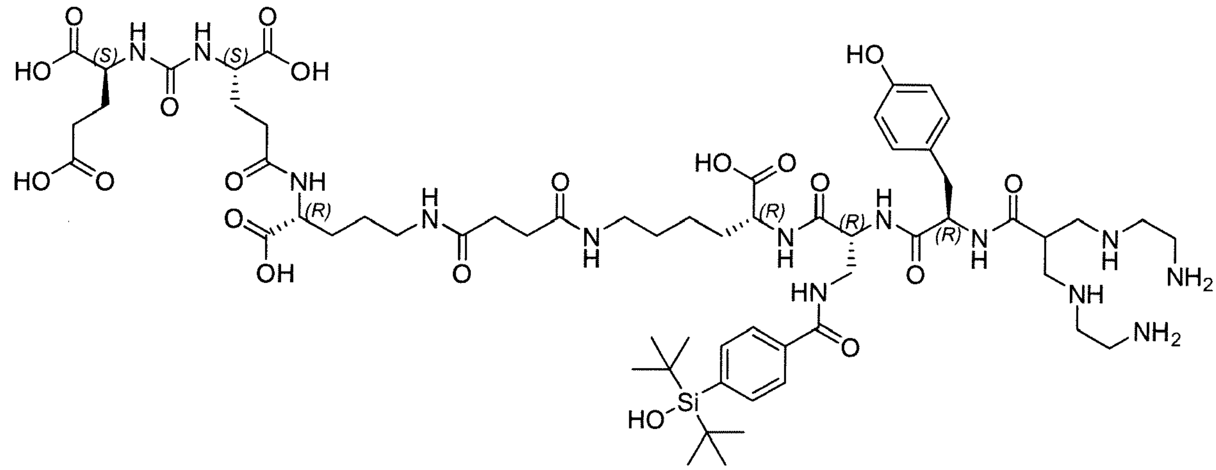

Chemical Formula: $C_{61}H_{97}N_{13}O_{20}Si$
Molecular Weight: 1360,60

N4-PSMA-32: RP-HPLC (10-60% B in 15 min): $t_R$ = 9.2 min, K' = 3.68. Calculated monoisotopic mass ($C_{61}H_{97}N_{13}O_{20}Si$): 1359.7; found: m/z (ESI) = 681.6 $[M+2H]^{2+}$, 454.7 $[M+3H]^{3+}$.

N4-PSMA-33:

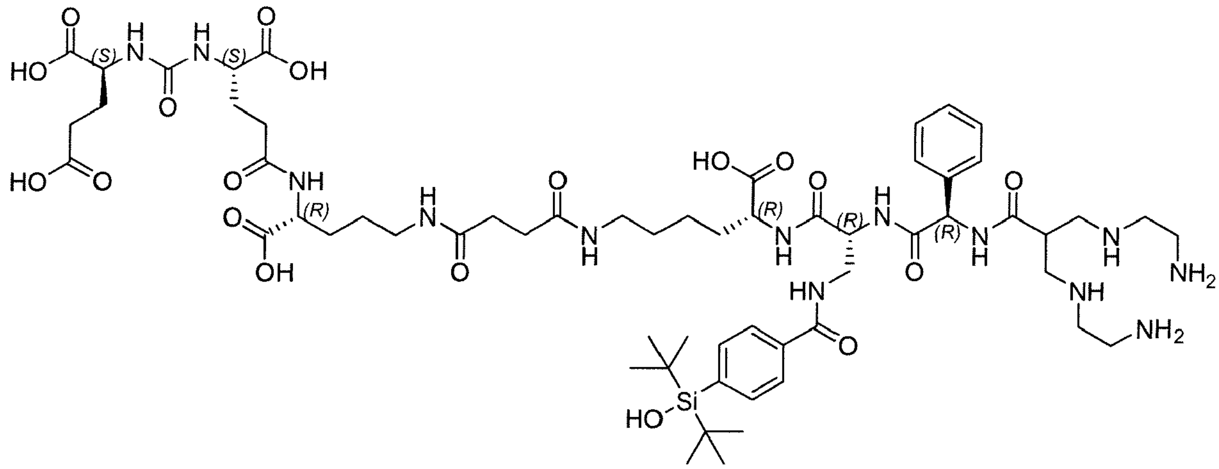

Chemical Formula: $C_{60}H_{95}N_{13}O_{19}Si$
Molecular Weight: 1330,58

N4-PSMA-33: RP-HPLC (10-60% B in 15 min): $t_R$ = 9.4 min, K' = 3.76. Calculated monoisotopic mass ($C_{60}H_{95}N_{13}O_{19}Si$): 1329.7; found: m/z (ESI) = 665.0 $[M+2H]^{2+}$, 443.6 $[M+3H]^{3+}$.

PSMA-I&S (reference compound):

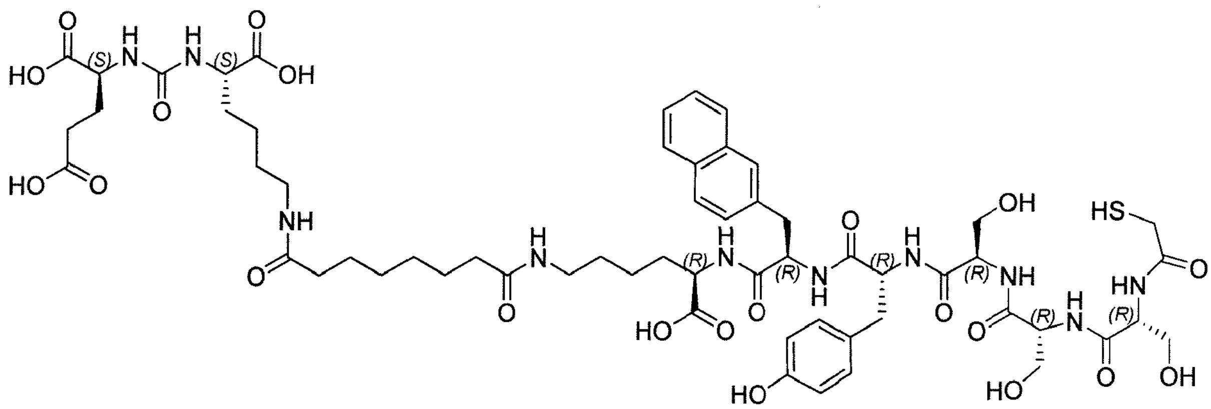

Chemical Formula: $C_{59}H_{82}N_{10}O_{21}S$
Molecular Weight: 1299,41

PSMA-I&S: RP-HPLC (10-70% B in 15 min): $t_R$ = 8.8 min, K' = 5.02. Calculated monoisotopic mass ($C_{59}H_{82}N_{10}O_{21}S$): 1298.5; found: m/z (ESI) = 1299.0 $[M+H]^+$, 650.6 $[M+2H]^{2+}$.

*IBA-KuE:*

For the synthesis of IBA-KuE, the protected binding motive (O*t*Bu)KuE(O*t*Bu)$_2$ was synthesized as previously described [28]. 4-iodo-benzoic acid (6.1 mg, 24.6 μmol, 1.2 eq.) was preactivated by addition of TBTU (7.9 mg, 24.6 μmol, 1.2 eq.), HOAt (3.3 mg, 24.6 μmol, 1.2 eq.) and DIPEA (12.9 μL, 73.8 μmol, 3.6 eq.) in DMF (1 mL). After 5 min of pre-activation at room temperature, (O*t*Bu)KuE(O*t*Bu)$_2$ (10.0 mg, 20.5 μmol, 1.0 eq.) in DMF (2 mL) was added and the solution was stirred overnight (21 h) at room temperature. The solvent was evaporated and upon addition of TFA (+2.5% TIPS, +2.5% $H_2O$) the solution was stirred for 1 h. TFA was evaporated and the crude product dissolved in DMF. After purification by semipreparative RP-HPLC (30-45% B in 20 min) the product was obtained as colorless, amorphous solid (48%).

RP-HPLC (20-40% B in 20 min): $t_R$ = 11.0 min, K' = 7.68. Calculated monoisotopic mass ($C_{19}H_{24}IN_3O_8$): 549.1; found: m/z (ESI) = 550.2 $[M+H]^+$.

**Radiolabeling**

*Radiolabeling of N4-PSMA ligands*

$^{99m}$Tc-Labeling of N4-PSMA ligands was carried out by addition of 1 nmol peptide precursor (0.5 mm in DMSO) to a mixture of 0.05 M $Na_2HPO_4$ (12.5 μL, in Tracepur®-water, pH 9.25) and 0.1 M disodium citrate sesquihydrate (1.5 μL, in Tracepur®-water) in saline. After addition of a freshly prepared solution of $SnCl_2$(2.5 μL, 1 mg/mL in ethanol), $[^{99m}Tc]TcO_4^-$ (40 MBq/nmol) in saline was added and the labeling solution (final volume 250 μL) was heated to 95°C for 15 min. Subsequently, 10 μL of 1 M sodium ascorbate (in PBS) was added and quality control was performed using radio-TLC and radio-RP-HPLC (UV-detection at 220 nm).

*Radiosynthesis of [$^{99m}$Tc]Tc-N4-PSMA-12 at patient scale*

In a 10 mL glass vial, 15 nmol N4-PSMA-12 (20 μg, 0.5 mm in DMSO) were added to a mixture of 0.05 M $Na_2HPO_4$ (250 μL, in TP-water, pH 9.25) and 0.1 M disodium citrate sesquihydrate (30 μL, in TP-water) in saline. After addition of a freshly prepared solution of $SnCl_2$ (10 μL, 1 mg/mL in ethanol), $[^{99m}Tc]TcO_4^-$ in saline was added and the labeling solution (final volume 2-5 mL) was heated to 95°C for 15 min. The labeling solution was left to cool for 10 min and, subsequently, quality control was performed using radio-TLC and radio-RP-HPLC.

*Radiosynthesis of [$^{9m}$Tc]Tc-PSMA-I&S (reference compound)*

Labeling of PSMA-I&S was carried out using 2 nmol of peptide precursor in a kit formulation as described by Robu et al [15]. After addition of $[^{9m}Tc]TcO_4^-$ (40 MBq/nmol) in 500 μL saline the solution was heated to 95°C for 20 min. Subsequently, 10 μL of 1 M sodium ascorbate (PBS) was added and quality control was performed using radio-TLC and radio-RP-HPLC.

*Radioiodination of [$^{25}$I]IBA-KuE*

The synthesis of the protected stannyl-precursor, radioiodination and deprotection yielding [$^{125}$I]IBA-KuE was carried out as previously described [28]. Purification of the crude labelling product was performed using preparative radio-RP-HPLC with a gradient of 20-40% B in 20 min.

*Analytical data of PSMA inhibitors labeled with technetium-99m or iodine-125*

[$^{99m}$Tc]Tc-N4-PSMA-12: radio-*RP*-HPLC (10-70% B in 15 min): $t_R$ = 9.1 min, K' = 6.36.
[$^{99m}$Tc]Tc-N4-PSMA-13: radio-*RP*-HPLC (10-70% B in 15 min): $t_R$ = 10.0 min, K' = 6.98.
[$^{99m}$Tc]Tc-N4-PSMA-21: radio-*RP*-HPLC (10-70% B in 15 min): $t_R$ = 8.6 min, K' = 5.99.
[$^{99m}$Tc]Tc-PSMA-I&S: radio-*RP*-HPLC (10-70% B in 15 min): $t_R$ = 8.2 min, K' = 5.76.
[$^{125}$I]IBA-KuE: radio-*RP*-HPLC (20-40% B in 20 min): $t_R$ = 11.0 min, K' = 7.68.

***Lipophilicity and binding to human plasma***

The lipophilicity of $^{99m}$Tc-labeled PSMA ligands, expressed as distribution coefficient (log$D_{7.4}$), was determined using the shake-flask method. A solution of approximately 1 MBq of radioligand in 1 mL of a 1:1 mixture of PBS (pH = 7.4) and n-octanol (n = 8) was vortexed vigorously for 3 min. After centrifugation at 9000 rpm for 5 min aliquots of both phases (200 $\mu$L n-octanol, 50 $\mu$L PBS) were collected and the activity was quantified in a $\gamma$-counter. log$D_{7.4}$ values were calculated as decadic logarithm of the activity concentration ratio between the n-octanol phase and the aqueous phase. Data are given as mean $\pm$ standard deviation (SD). Binding to human plasma was determined by incubation of radioligands in human plasma (2 nM concentration, 37 °C for 30 min) and subsequent ultrafiltration (250 $\mu$L aliquots, 3200 rpm, 40 min) in Centrifree® ultrafiltration devices (Merck Millipore, Cork, Ireland). The fraction bound to human plasma proteins was calculated as the ratio of unfiltered activity and the total activity in the ultrafiltration device. Measurements were performed in three independent experiments, each with two replicates (n = 6, data are given as mean $\pm$ SD). All values were corrected for non-specific binding (control experiments in PBS).

***Determination of human serum albumin (HSA) binding by high performance affinity chromatography (HPAC)***

HSA binding of the uncomplexed PSMA ligands was determined according to a previously published procedure via HPLC (Valko, K.; Nunhuck, S.; Bevan, C.; Abraham, M. H.; Reynolds, D. P., Fast gradient HPLC method to determine compounds binding to human serum albumin. Relationships with octanol/water and immobilized artificial membrane lipophilicity. J Pharm Sci. 2003, 92, 2236-2248). A Chiralpak HSA column (50 $\times$ 3 mm, 5 $\mu$m, H13H-2433, Daicel, Tokyo, Japan) was used at a constant flow rate of 0.5 mL/min at rt. Mobile phase A was a freshly prepared 50 mM aqueous solution of $NH_4OAc$ (pH 6.9) and mobile phase B was isopropanol (HPLC grade, VWR). The applied gradient for all experiments was 100% A (0 to 3 min), followed by 80% A (3 to 40 min). Prior to the experiment, the column was calibrated using nine reference substances with a HSA binding, known from literature, in the range of 13 to 99% (Valko, K.; Nunhuck, S.; Bevan, C.; Abraham, M. H.; Reynolds, D. P., J Pharm Sci. 2003, 92, 2236-2248; Yamazaki, K.; Kanaoka, M., Computational prediction of the plasma protein-binding percent of diverse pharmaceutical compounds. J Pharm Sci. 2004, 93, 1480-1494). All substances, including the examined PSMA ligands, were dissolved in a 1:1 mixture (v/v) of isopropanol and a 50 mM aqueous solution of $NH_4OAc$ (pH 6.9) with a final concentration of 0.5 mg/mL. Non-linear regression was established with the OriginPro 2016G software (Northampton, United States).

*Determinations of Affinities ($IC_{50}$)* ***and Internalization Studies***

Competitive binding studies were carried out in analogy to a previously reported procedure [28]. As a modification the non-radiolabeled standard competitor (((S)-1-carboxy-5-(4-(iodo)benzamido)pentyl)carbamoyl)-L-glutamic acid (IBA-KuE) was applied in increasing concentrations ($10^{-5}$ - $10^{-11}$ M/well, n = 3 each) whereas the novel PSMA-binding compounds of interest were applied as $^{99m}$Tc-labeled radioligands (0.2 nM/well). In this inversed experimental approach, higher values correspond to higher affinities and are referred to as *inverse* $IC_{50}$. Poly-L-lysine coated 24-well plates were used (n = 3). Data are given as mean $\pm$ SD. To determine the cellular uptake of the $^{99m}$Tc-labeled PSMA ligands into LNCaP cells by means of PSMA-mediated internalization at 1 h, a previously reported protocol was applied with assay concentrations of 1.0 nM and 0.2 nM for $^{99m}$Tc-labeled PSMA ligands and the reference compound [$^{125}$I]IBA-KuE, respectively [28]. Data are corrected for non-specific binding and normalized to the specific internalization of the reference. Results are given as mean $\pm$ SD.

### *In Vivo Experiments*

All animal experiments were conducted in accordance with general animal welfare regulations in Germany (German animal protection act, in the edition of the announcement, dated May 18th, 2006, as amended by Article 280 of June 19th 2020, approval no. ROB-55.2-1-2532.Vet_02-18-109 by the General Administration of Upper Bavaria) and the institutional guidelines for the care and use of animals. Male CB17-SCID mice were purchased from Charles River (Sulzfeld, Germany) and arrived at the in-house animal facility to acclimate at least 1 week before the start of the experiment. Tumor xenografts were established by subcutaneous inoculation of LNCaP cells (approx. $2 \times 10^7$ cells in 200 $\mu$L of a 1:1 mixture of Cultrex BME (R&D Systems, Minneapolis, United States) and DMEM/Ham's F-12) onto the right shoulder of 6-8 weeks old male CB17-SCID mice. The animals were used for experiments when tumors had grown to a size of 5-10 mm in diameter. Criteria for the exclusion of animals from the experiment were weight loss higher than 20%, tumor size above 1.5 $cm^3$, ulceration of the tumor, respiratory distress or change of behavior. These criteria did not apply to any mouse. Neither randomisation nor blinding was applied in the allocation of the experiments. Quarterly health monitoring was performed according to the FELASA recommendations.

*Biodistribution Studies.* The $^{99m}$Tc-labeled radioligands (2.7 $\pm$ 0.7 MBq, 82 $\pm$ 20 pmol) were injected into a lateral tail vein of LNCaP-tumor bearing mice (n = 4-5) under isoflurane anesthesia. Animals were sacrificed at 6 h post injection (p.i.) by carbon dioxide inhalation and blood withdrawal via cardiac puncture. Blood and tissues of interest were collected, weighed and the activity measured in a $\gamma$-counter. Radioligand uptake is given in percent of the injected dose per gram of tissue (% ID/g) and results are presented as mean $\pm$ SD.

In line with the above, the biodistribution study of [$^{99m}$Tc]Tc-N4-PSMA-21 was carried out with n = 5 animals ("data set I"). All animals were healthy and did not show any signs of pain, distress or change in behavior throughout the experiment. However, gross deviations in the kidney uptake and elevated tumor uptake were observed for one particular animal (mouse #3) as compared to the other four animals. Kidney uptake of mouse #3 (36.17 %ID/g) exceeded the value obtained from the other four animals (4.58 $\pm$ 1.44 %ID/g, mean $\pm$ SD) 7.9-fold or by more than 20 standard deviations. In addition, tumor uptake of mouse #3 (21.92 %ID/g) exceeded the value obtained from the other four animals (11.02 $\pm$ 2.22 %ID/g, mean $\pm$ SD) 2.0-fold or by more than 4 standard deviations Due to the uncertainty whether these huge deviations arise from an experimental error or might be related to strongly deviating metabolic processes e.g. abnormal kidney function in mouse #3, the study primarily presents and discusses the data set based on the other four animals (n = 4, "data set II"). A thorough evaluation and comparison was, however, also carried out including mouse #3. For both, "data set I" (n = 5, including mouse #3) and "data set II" (n = 4, without mouse #3) kidney uptake of [$^{99m}$Tc]Tc-N4-PSMA-21 was significantly reduced compared to [$^{99m}$Tc]Tc-PSMA-I&S (both $P < 0.001$) but not statistically different from other $^{99m}$Tc-labeled N4-PSMA-ligands ($P > 0.92$ and $P > 0.81$ for "data set I" and "data set II", respectively). Although mouse #3 also showed an elevated tumor uptake (see above), no statistically significant differences in tumor uptake among the $^{99m}$Tc-labeled PSMA-ligands evaluated herein were found, regardless of whether "data set I" ($P > 0.69$) or "data set II" ($P > 0.27$) was compared to the biodistribution data of the other radioligands.

*$\mu$SPECT/CT Imaging.* Static imaging of sacrificed animals was performed on a VECTor4 small-animal SPECT/PET/CT/OI scanner from MILabs (Utrecht, Netherlands) directly after blood collection with an acquisition time of 45 min using an HE-GP-RM collimator and a step-wise multiplanar bed movement via MILabs acquisition software (v11.00 and v12.26). Imaging data was reconstructed using MILabs-Reconstruction software (v12.00) and image analysis was performed with PMOD4.0 (PMOD technologies LLC, Zurich, Switzerland). Animals were subjected to biodistribution studies after imaging.

### *Data analysis*

Acquired data was statistically analyzed performing a one-way analysis of variances (ANOVA) followed by a Tukey's multiple comparison post-test using OriginPro software (version 9.7) from OriginLab Corporation (Northampton, United States). Pairwise statistical comparisons were performed applying the two-sample student's t-test in Microsoft Excel (Redmond, United States). Acquired P-values of < 0.05 were considered statistically significant.

## Results

### *Synthesis and Radiolabeling*

The novel PSMA ligands were synthesized using a mixed solution/solid phase synthetic approach and obtained with chemical purity of >98% in yields of 29%, 25% and 21%, respectively. Compound identity was confirmed by mass spectrometry. Radiolabeling with [$^{99m}$Tc]$TcO_4^-$ resulted in radiochemical purities (RCP) of >95% as determined by radio-TLC and radio-RP-HPLC. Up-scaling of the reported labeling protocol for patient-scale production of [$^{99m}$Tc]Tc-N4-PSMA-12 was found to be feasible, however proportionally reduced amounts of stannous chloride were applied to

prevent the formation of colloidal technetium-species. Under consideration of requirements of a clinical workflow for routine radiosynthesis, labeling of N4-PSMA-12 (20 μg, 15 nmol) with activities of 194-810 MBq (553 ± 187 MBq, mean ± SD, n = 10) reproducibly yielded the desired radioligand with a RCP of 98.5 ± 0.6% (range, 97.6 - 99.2%, n = 10). Detailed information on single radiolabelings are provided in the following table 1.

**Table 1:** Detailed data on $^{99m}$Tc-labeling of N4-PSMA-12 at patient scale. The volume of the labeling solution (V), the applied activity (A) as well as the amount of free [$^{99m}$Tc]$TcO_4^-$ and colloidal technetium-99m, as determined via radio-TLC, and the resulting radio chemical purity (RCP) are given for single labeling experiments. Mean values and standard deviation (SD, n = 10) are given for the whole data set.

| entry | V [mL] | A [MBq] | Free [$^{99m}$Tc]$TcO_4^-$ [%] | Colloidal Tc-99m [%] | RCP [%] |
|---|---|---|---|---|---|
| 1 | 5.00 | 194 | 0.21 | 0.85 | 98.94 |
| 2 | 5.00 | 810 | 0.40 | 0.44 | 99.16 |
| 3 | 5.00 | 542 | 0.24 | 2.21 | 97.55 |
| 4 | 5.00 | 502 | 0.17 | 1.98 | 97.85 |
| 5 | 5.00 | 760 | 0.30 | 1.78 | 97.92 |
| 6 | 3.18 | 634 | 0.23 | 1.12 | 98.65 |
| 7 | 2.66 | 470 | 0.23 | 1.02 | 98.75 |
| 8 | 3.02 | 388 | 0.31 | 0.64 | 99.05 |
| 9 | 2.00 | 727 | 0.97 | 0.73 | 98.30 |
| 10 | 4.00 | 501 | 0.28 | 1.31 | 98.41 |
| mean | 3.99 | 553 | 0.33 | 1.21 | 98.46 |
| SD | 1.18 | 187 | 0.23 | 0.60 | 0.55 |

### *In Vitro* Characterization

Results of the *in vitro* characterization of [$^{99m}$Tc]Tc-labeled N4-PSMA ligands and the reference [$^{99m}$Tc]Tc-PSMA-I&S are summarized in figure 1 and in the following tables 2a and 2b. PSMA affinity was assessed via determination of the inverse $IC_{50}$ ($IC_{50,inv}$). Irrespective of the variable amino acid, all radioligands showed high PSMA affinity displayed by low nanomolar $IC_{50,inv}$. values (range, 10.0 - 11.8 nm) with no statistically significant difference (P > 0.66). In contrast, PSMA mediated internalization, expressed as percentage of the reference compound [$^{125}$I]IBA-KuE, was significantly influenced by the variable amino acid. [$^{99m}$Tc]Tc-N4-PSMA-12 (311 ± 16%) exhibited 1.3-fold higher internalization than [$^{99m}$Tc]Tc-PSMA-I&S (240 ± 13%), and an even 1.9- and 1.7-fold higher value than [$^{99m}$Tc]Tc-N4-PSMA-13 (164 ± 15%) and [$^{99m}$Tc]Tc-N4-PSMA-21 (180 ± 4%), respectively. In comparison with [$^{99m}$Tc]Tc-PSMA-I&S, all novel [$^{99m}$Tc]Tc-N4-PSMA compounds showed increased hydrophilicity, expressed by the distribution coefficient (log$D_{7.4}$). Within the group of N4-bearing radioligands, highest lipophilicity was observed for [$^{99m}$Tc]Tc-N4-PSMA-13 (log$D_{7.4}$ = -2.78 ± 0.05), comprising an aromatic D-Phe residue, followed by [$^{99m}$Tc]Tc-N4-PSMA-21 (log$D_{7.4}$ = -3.13 ± 0.05, D-(4-$NH_2$)-Phe) and [$^{99m}$Tc]Tc-N4-PSMA-12 (log$D_{7.4}$ = -3.35 ± 0.05, D-Glu). Compared to [$^{99m}$Tc]Tc-PSMA-I&S, binding to human plasma was significantly reduced for all [$^{99m}$Tc]Tc-N4-PSMA tracers (94.4% vs 55.1 - 88.5%), and followed the same trend as described for lipophilicity. Thus, the highest hydrophilicity (log$D_{7.4}$ = -3.35 ± 0.05) and lowest binding to human plasma proteins (55.1 ± 2.5%) was both found for [$^{99m}$Tc]Tc-N4-PSMA-12 (P < 0.001 for both parameters).

**Table 2a:** Lipophilicity of three novel $^{99m}$Tc-labeled N4-PSMA compounds and $^{99m}$Tc-labeled PSMA-I&S expressed as partition coefficient ($logD_{7.4}$) using the n-octanol/PBS (pH 7.4) distribution system (n = 8), binding affinity towards PSMA (inverse $IC_{50}$ (nM), 1 h, 4°C, n = 3), PSMA-mediated internalization by LNCaP cells (1 h, 37°C, n = 3) as a percentage of the radiolabeled reference ([$^{125}$I]IBA)KuE) and plasma protein binding (PPB, determined by a ultrafiltration method, n = 6) of $^{99m}$Tc-labeled N4-PSMA compounds and [$^{99m}$Tc]Tc-PSMA-I&S as well as HSA-binding of uncomplexed compounds (determined by HPAC, n = 1).

| compound | [$^{99m}$Tc]Tc-N4-PSMA-12 | [$^{99m}$Tc]Tc-N4-PSMA-13 | [$^{99m}$Tc]Tc-N4-PSMA-21 | [$^{99m}$Tc]Tc-PSMA-I&S |
|---|---|---|---|---|
| log$D_{7.4}$ | -3.35 ± 0.05 | -2.78 ± 0.05 | -3.13 ± 0.05 | -2.61 ± 0.08 |
| $IC_{50, inv.}$ (nM) | 11.02 ± 2.80 | 9.98 ± 0.99 | 11.07 ± 1.42 | 11.78 ± 1.89 |

(continued)

| compound | $[^{99m}Tc]$Tc-N4-PSMA-12 | $[^{99m}Tc]$Tc-N4-PSMA-13 | $[^{99m}Tc]$Tc-N4-PSMA-21 | $[^{99m}Tc]$Tc-PSMA-I&S |
|---|---|---|---|---|
| internalization (% IBA-KuE) | 311 ±16 | 164± 15 | 180±4 | 240±13 |
| PPB (%) | 55.1 ± 2.5 | 88.5 ± 1.5 | 66.0 ± 2.3 | 94.4 ± 0.7 |
| HSA-binding (HPAC) (%) | 47.7 | 89.9 | 57.3 | 98.8 |

**Table 2b:** Lipophilicity of three novel $^{99m}Tc$-labeled N4-PSMA compounds expressed as partition coefficient ($logD_{7.4}$) using the n-octanol/PBS (pH 7.4) distribution system (n = 8), binding affinity towards PSMA (inverse $IC_{50}$ (nM), 1 h, 4°C, n = 3) and PSMA-mediated internalization by LNCaP cells (1 h, 37°C, n = 3) as a percentage of the radiolabeled reference ($[^{125}I]$IBA)KuE) of $^{99m}Tc$-labeled N4-PSMA compounds as well as HSA-binding of uncomplexed compounds (determined by HPAC, n = 1).

| compound | $[^{99m}Tc]$Tc-N4-PSMA-11 | $[^{99m}Tc]$Tc-N4-PSMA-32 | $[^{99m}Tc]$Tc-N4-PSMA-33 |
|---|---|---|---|
| $logD_{7.4}$ | -3.09 ± 0.01 | -2.87 ± 0.02 | -2.80 ± 0.07 |
| $IC_{50, inv.}$ (nM) | 6.06 ± 2.51 | 4.23 ± 0.29 | 5.29 ± 1.53 |
| internalization (% IBA-KuE) | 108 ± 4 | 71 ± 3 | 99 ± 7 |
| HSA-binding (HPAC) (%) | 52.6 | 87.2 | 88.1 |

***In Vivo Characterization***

*Biodistribution studies.* Comparative biodistribution studies in LNCaP tumor-bearing mice at 6 h p.i. were performed for all four radiotracers (figure 2, table 3).

Among the three N4-bearing radioligands, a similar distribution profile with high uptake in the tumor (11.0 - 13.0% ID/g), varying but moderate activity levels in the kidneys and efficient clearance from blood and background tissue was observed (see also μSPECT/CT-scans in figure 3). $[^{99m}Tc]$Tc-N4-PSMA-12 showed slightly higher kidney retention (12.3 ± 8.0% ID/g) than $[^{99m}Tc]$Tc-N4-PSMA-13 and $[^{99m}Tc]$Tc-N4-PSMA-21 (6.6 ± 4.8% ID/g and 4.6 ± 1.4% ID/g, respectively) which was, however, not statistically significant (P > 0.81). In clear contrast to the N4-PSMA radioligands, very high activity retention in the kidney was found for $[^{99m}Tc]$Tc-PSMA-I&S (191 ± 26% ID/g, >15-fold higher than that of $[^{99m}Tc]$Tc-N4-PSMA-12). Significantly higher activity retention after injection of $[^{99m}Tc]$Tc-PSMA-I&S at 6 h p.i. was also present in several other organs such as lungs, spleen, adrenals and parotid gland (P < 0.001 each). The higher activity uptake of $[^{99m}Tc]$Tc-PSMA-I&S in tumors, however, was less pronounced (15.6 ± 2.8% ID/g). The corresponding differences to the novel N4-PSMA radioligands were not statistically significant (P > 0.27). Efficient activity clearance from the blood pool was observed for all radioligands. The lowest blood activity level at 6 h p.i. was found for $[^{99m}Tc]$Tc-N4-PSMA-12 (0.0200 ± 0.0044% ID/g), which correlates with its low $logD_{7.4}$ and low PPB. Blood activity levels of $[^{99m}Tc]$Tc-N4-PSMA-13, $[^{99m}Tc]$Tc-N4-PSMA-21 and $[^{99m}Tc]$Tc-PSMA-I&S were increased by 1.6-, 5.4- and 3.6-fold, respectively, as compared to $[^{99m}Tc]$Tc-N4-PSMA-12.

*Tumor-to-background ratios (TBR).* As depicted in figure 4, $[^{99m}Tc]$Tc-N4-PSMA-12 showed the highest TBR among all radioligands throughout the majority of organs. It is noteworthy, that in direct comparison with $[^{99m}Tc]$Tc-PSMA-I&S, $[^{99m}Tc]$Tc-N4-PSMA-12 showed increased TBR for every analyzed organ and blood, thus demonstrating its notably superior clearance properties. In particular, a 3-fold T/blood-ratio (658 ± 147 vs 219 ± 62) and an even 20-fold T/kidney-ratio (1.64 ± 1.29 vs 0.08 ± 0.01) represent important advances towards optimized pharmacokinetics. Although less pronounced, improvements were also found for the TBR of $[^{99m}Tc]$Tc-N4-PSMA-13 and $[^{99m}Tc]$Tc-N4-PSMA-21. A tabular overview of TBR is provided in the following table 4.

**Table 3**: Numeric data of the biodistribution of $^{99m}$Tc-labeled N4-PSMA compounds and $^{99m}$Tc-labeled PSMA-I&S at 6 h p.i. in male LNCaP tumor-bearing CB17-SCID mice. Data are expressed as percentage of the injected dose per gram (% ID/g), mean ± standard deviation (SD, n = 4-5).

| | [$^{99m}$Tc]Tc-N4-PSMA-12 (n = 5) | | [$^{99m}$Tc]Tc-N4-PSMA-13 (n=5) | | [$^{99m}$Tc]Tc-N4-PSMA-21 (n=4) | | [$^{99m}$Tc]Tc-PSMA-I&S (n = 4) | |
|---|---|---|---|---|---|---|---|---|
| uptake in % iD/g | mean | SD | mean | SD | mean | SD | mean | SD |
| blood | 0.0200 | 0.0044 | 0.0320 | 0.0100 | 0.1074 | 0.0623 | 0.0728 | 0.0072 |
| heart | 0.0274 | 0.0083 | 0.0456 | 0.0090 | 0.0568 | 0.0128 | 0.1246 | 0.0252 |
| lung | 0.0613 | 0.0177 | 0.1108 | 0.0186 | 0.1250 | 0.0578 | 1.0378 | 0.0612 |
| liver | 0.1796 | 0.0890 | 0.2618 | 0.0556 | 0.5752 | 0.3652 | 0.2215 | 0.0234 |
| spleen | 0.8928 | 0.5700 | 0.6314 | 0.1819 | 1.3064 | 0.7969 | 31.8636 | 6.0507 |
| pancreas | 0.0206 | 0.0031 | 0.0270 | 0.0053 | 0.0335 | 0.0099 | 0.4040 | 0.0639 |
| stomach | 0.0988 | 0.0506 | 0.1348 | 0.0441 | 0.1458 | 0.0956 | 0.2306 | 0.1018 |
| intestine | 0.5391 | 0.4453 | 0.2661 | 0.1637 | 1.1034 | 1.7380 | 0.6415 | 0.3960 |
| kidney | 12.2924 | 8.0491 | 6.5974 | 4.7823 | 4.5811 | 1.4363 | 191.560 | 25.7140 |
| adrenals | 0.6570 | 0.2747 | 0.5170 | 0.0912 | 0.5759 | 0.1443 | 13.5354 | 2.9883 |
| muscle | 0.0110 | 0.0041 | 0.0127 | 0.0018 | 0.0153 | 0.0027 | 0.1020 | 0.0290 |
| bone | 0.0365 | 0.0136 | 0.0862 | 0.0139 | 0.0828 | 0.0778 | 0.0907 | 0.0157 |
| tumor | 13.0231 | 3.8549 | 12.5347 | 4.0835 | 11.0155 | 2.2227 | 15.6183 | 2.8033 |
| parotid gland | 0.0804 | 0.0339 | 0.1056 | 0.0262 | 0.1105 | 0.0199 | 1.7348 | 0.5573 |
| submandibular gland | 0.1450 | 0.0431 | 0.3069 | 0.0642 | 0.3009 | 0.2151 | 0.6196 | 0.0695 |

**Table 4:** Tumor-to-background ratios of $^{99m}$Tc-labeled N4-PSMA compounds and $^{99m}$Tc-labeled PSMA-I&S at 6 h p.i. in male LNCaP tumor-bearing CB17-SCID mice. Data are given as mean ± standard deviation (SD, n = 4-5). Mean values were determined from tumor-to-organ ratios calculated for individual animals.

| tumor-to-background | [$^{99m}$Tc]Tc-N4-PSMA-12 (n = 5) | | [$^{99m}$Tc]Tc-N4-PSMA-13 (n = 5) | | [$^{99m}$Tc]Tc-N4-PSMA-21 (n = 4) | | [$^{99m}$Tc]Tc-PSMA-I&S (n = 4) | |
|---|---|---|---|---|---|---|---|---|
| ratio | mean | SD | mean | SD | mean | SD | mean | SD |
| blood | 657.74 | 146.75 | 409.29 | 158.60 | 125.55 | 62.05 | 218.97 | 62.09 |
| heart | 484.90 | 122.64 | 276.38 | 87.34 | 201.50 | 62.05 | 127.22 | 20.53 |
| lung | 216.68 | 56.82 | 113.30 | 35.32 | 99.35 | 36.86 | 15.01 | 2.29 |
| liver | 79.92 | 30.33 | 50.17 | 21.50 | 23.75 | 10.51 | 71.64 | 19.02 |
| spleen | 17.89 | 9.02 | 20.57 | 7.45 | 10.32 | 4.39 | 0.49 | 0.06 |
| pancreas | 637.21 | 201.56 | 496.73 | 247.35 | 350.74 | 126.56 | 40.19 | 13.36 |
| stomach | 158.87 | 78.55 | 101.82 | 47.12 | 114.49 | 101.37 | 76.24 | 27.21 |
| intestine | 57.76 | 60.23 | 64.62 | 39.59 | 52.29 | 48.36 | 38.15 | 31.76 |
| kidney | 1.64 | 1.29 | 2.45 | 1.45 | 2.57 | 0.77 | 0.08 | 0.01 |
| adrenals | 22.15 | 9.04 | 24.50 | 8.36 | 19.85 | 4.82 | 1.24 | 0.52 |
| muscle | 1265.34 | 486.05 | 1013.12 | 379.65 | 736.44 | 193.04 | 159.28 | 39.53 |
| bone | 384.40 | 158.05 | 142.94 | 32.47 | 207.38 | 115.07 | 178.36 | 51.53 |
| parotid gland | 177.38 | 73.36 | 119.35 | 34.53 | 101.59 | 24.83 | 9.87 | 3.90 |
| submandibular gland | 90.45 | 15.58 | 41.60 | 14.26 | 50.80 | 32.33 | 25.44 | 5.00 |

**Discussion**

With the N4-PSMA ligand design a versatile structural platform is provided that ensures high PSMA affinity and reliable complexation of radioisotopes such as technetium-99m while also allowing for flexible modifications to adjust the pharmacokinetic profile of the entire ligand. To retain high affinity, while ensuring low lipophilicity of the new tracers,

a less lipophilic SiOH-moiety (formally a hydrolyzed SiFA) was used. For complexation of the radioisotope, a tetraamine chelator was chosen, as this chelating system exhibits excellent *in vivo* stability and confers high hydrophilicity to peptidic radioligands [29, 30]. A further advantage over $N_3S$-based chelating systems such as mercaptoacetyltriserine is the absence of chemically reactive thiol-groups which might limit the shelf-live of radioligand precursors, an oftentimes neglected aspect in early radioligand development. Finally, the incorporation of a variable amino acid afforded novel ligands with distinct properties *in vitro* and *in vivo.*

Slow whole-body clearance and in part hepatobiliary excretion of [$^{99m}$Tc]Tc-PSMA-I&S is assumed to be caused by high PPB and increased lipophilicity compared to other PSMA-addressing radiometal chelates [15]. Therefore, a main focus in the design of the novel ligands was set on reduced lipophilicity and PPB. These objectives were met by the N4-PSMA compounds. The intrinsic lipophilicity of the variable amino acid (logP: Phe > (4-$NH_2$)-Phe > Glu) [31] translated into similar trends for lipophilicity and PPB of the corresponding radioligands (log$D_{7.4}$ and PPB: [$^{99m}$Tc]Tc-PSMA-I&S > [$^{99m}$Tc]Tc-N4-PSMA-13 > [$^{99m}$Tc]Tc-N4-PSMA-21 > [$^{99m}$Tc]Tc-N4-PSMA-12). Especially [$^{99m}$Tc]Tc-N4-PSMA-12 combines a favorable lipophilicity, comparable to diagnostic rhPSMA compounds [23], and a PPB that is significantly lower than for [$^{99m}$Tc]Tc-PSMA-I&S, [$^{68}$Ga]Ga/[$^{177}$Lu]Lu-PSMA-I&F [32] or [$^{99m}$Tc]Tc-EuK-($SO_3$)Cy5-$mas_3$ [25] (all ligands developed for PSMA-guided surgery) and comparable to fast-clearing [$^{177}$Lu]Lu-PSMA-617 [33]. In agreement with many prior studies [25-27], these findings underline how the pharmacokinetically relevant properties of a radioligand can be shaped by thoughtful structural modifications.

Even though [$^{99m}$Tc]Tc-PSMA-I&S showed favorable dosimetry in patients [21] and application in RGS suggests superiority over conventional salvage surgery [17], we have to admit that [$^{99m}$Tc]Tc-PSMA-I&S is not yet the optimal radioligand for RGS. This is exemplified by a study among 31 patients by *Maurer et al.,* who reported successful resection of all lesions detected on prior PSMA-PET and even additional lesions as small as 3 mm [13]. However, the same study revealed that in 12 of 86 resected tissue specimens that were classified PSMA-negative according to $\gamma$-probe measurements, histochemical analysis discovered previously unidentified metastatic lesions.

The obvious need for higher sensitivity in RGS is, from a radiopharmaceutical perspective, a need for an optimized radioactive probe providing higher TBR during surgery. Thus, to assess TBR of our novel $^{99m}$Tc-labeled N4-PSMA ligands, biodistribution studies at 6 h p.i. were performed. This rather long distribution time comes with the limitation of scarce comparability with literature, where mostly time-points of 1 h or 4 h were investigated. However, RGS is performed around 20-24 h p.i. in patients [13, 19, 34] and, based on the rule of thumb of approximately 4-fold faster metabolism in mice compared to men, we chose a distribution time of 6 h to simulate TBR at the time of surgery as accurately as possible. In that context, our preclinical findings on the *in vivo* performance of [$^{99m}$Tc]Tc-N4-PSMA ligands represent a remarkable development. While similar high uptake in LNCaP-xenografts was observed at 6 h p.i., the clearance of the novel ligands from most background tissue was significantly improved compared to [$^{99m}$Tc]Tc-PSMA-I&S. In addition, drastically and favorably reduced kidney retention compared to [$^{99m}$Tc]Tc-PSMA-I&S was found to be a common feature of the new ligands, which can probably be attributed to their shared molecular scaffold derived from rhPSMA-compounds [23, 24], while being pronounced by the use of the hydrolyzed SiFA moiety. This finding is of particular interest, as high renal activity accumulation may interfere with accurate lesion detection during RGS [34].

To an even greater extend, incomplete clearance of [$^{99m}$Tc]Tc-PSMA-I&S from the blood pool and thus a suboptimal T/blood-ratio can affect the accuracy of RGS [15]. In this regard, the decreased blood activity at 6 h p.i. represents another distinct advantage of preferred PSMA ligand compounds in accordance with the invention, in particular the 3.6 fold decrease observed for [$^{99m}$Tc]Tc-N4-PSMA-12. Compared to [$^{99m}$Tc]Tc-N4-PSMA-12, slightly higher uptake in blood and some background organs such as heart, lung, liver, parotid gland and submandibular gland were observed for [$^{99m}$Tc]Tc-N4-PSMA-13 and [$^{99m}$Tc]Tc-N4-PSMA-21, which can be attributed to the incorporation of the aromatic amino acids D-Phe and D-(4-$NH_2$)Phe conferring higher lipophilicity and plasma protein binding to these compounds compared to [$^{99m}$Tc]Tc-N4-PSMA-12 comprising the negatively charged amino acid D-Glu. A further possible limitation of [$^{99m}$Tc]Tc-PSMA-I&S is background activity in the bowel hampering the detection of lesions with low signal strength during surgery. In this regard, when completely ignoring the significantly improved T/kidney ratio of [$^{99m}$Tc]Tc-N4-PSMA-12 and when taking into account only the hepatic and intestinal uptake of [$^{99m}$Tc]Tc-PSMA-I&S and [$^{99m}$Tc]Tc-N4-PSMA-12 (no statistically significant difference observed, $P > 0.19$ and $P > 0.36$, respectively), we would expect at least similar performance of [$^{99m}$Tc]Tc-PSMA-I&S and [$^{99m}$Tc]Tc-N4-PSMA-12 in men. Beyond that, the finding that TBR of blood and all analyzed organs (see figure 4) obtained with [$^{99m}$Tc]Tc-N4-PSMA-12 are higher than that of [$^{99m}$Tc]Tc-PSMA-I&S clearly demonstrates the superior pharmacokinetic profile of the radioligand in accordance with the invention.

To facilitate future clinical translation, we developed a protocol for GMP-production of [$^{99m}$Tc]Tc-N4-PSMA-12 (see figure 5). As the simple, reliable, one-step process complies with basic nuclear medicine infrastructure and established clinical workflows and procedures, it provides an important basis for widespread availability of [$^{99m}$Tc]Tc-N4-PSMA-12 and may contribute to minimize the logistic hurdle for a first clinical application.

**Abbreviations**

HPLC: high performance liquid chromatography; IBA-KuE: (((S)-1-carboxy-5-(4-(iodo)benzamido)pentyl)carbamoyl)-L-glutamic acid; LNM: lymph node metastases; mCRPC: metastatic castration resistant prostate cancer; N4: tetraamine / 6-carboxy-1,4,8,11-tetraazaundecane; PBS: phosphate buffered saline; PCa: prostate cancer; PET: positron emission tomography; PPB: plasma protein binding; PSMA: prostate specific membrane antigen; RCP: radiochemical purity; RGS: radioguided surgery; RP: reversed phase; SD: standard deviation; SiFA: silicon fluoride acceptor; sLND: sentinel lymph node dissection; SPECT: single photon emission computed tomography; TBR: tumor-to-background ratio; TLC: thin layer chromatography; UV: ultra-violet.

**Figure 1:** *In vitro* characterization of [$^{99m}$Tc]Tc-N4-PSMA-12, [$^{99m}$Tc]Tc-N4-PSMA-13, [$^{99m}$Tc]Tc-N4-PSMA-21 and [$^{99m}$Tc]Tc-PSMA-I&S: A) Binding affinity to PSMA ($IC_{50,inv.}$ (nM), 1 h, 4°C, n = 3); B) PSMA-mediated internalization (1 h, 37°C, (% of the reference [$^{125}$I]IBA-KuE), n = 3); C) lipophilicity expressed as log$D_{7.4}$ (n-octanol/PBS, pH 7.4, n = 8); D) binding to human plasma (PPB) (incubation at 37°C for 30 min, determination via ultrafiltration (%), n = 6).
**Figure 2:** *Ex vivo* biodistribution data of $^{99m}$Tc-labeled N4-PSMA derivatives and [$^{99m}$Tc]Tc-PSMA-I&S at 6 h p.i. in male LNCaP tumor-bearing CB17-SCID mice. Data are expressed as a percentage of the injected dose per gram (% ID/g), mean ± standard deviation (n = 4-5). gl.: gland; submand.: submandibular.
**Figure 3**: Static µSPECT/CT images (maximum intensity projections) of $^{99m}$Tc-labeled N4-PSMA-derivatives and [$^{99m}$Tc]Tc-PSMA-I&S in LNCaP tumor-bearing mice. Animals were sacrificed at 6 h p.i. and imaged directly after blood collection for 45 min on a VECTor4 small-animal SPECT/PET/OI/CT. Tracer uptake in tumor and kidneys (in percent of the injected dose/gram, (% ID/g)) was determined from subsequent biodistribution studies.
**Figure 4:** Tumor-to-background ratios (TBR) of $^{99m}$Tc-labeled N4-PSMA compounds and [$^{99m}$Tc]Tc-PSMA-I&S at 6 h p.i. in male LNCaP tumor-bearing CB17-SCID mice. Data are given as mean ± standard deviation (n = 4-5). Mean values were determined from TBR calculated for individual animals. gl.: gland; submand.: submandibular.
**Figure 5:** Suggested work-flow for clinical production of [$^{99m}$Tc]Tc-N4-PSMA-12. Patient scale production is completed within 35 min post generator elution under non-optimized laboratory conditions. Optimization for clinical routine synthesis will further reduce the time of production. RT: room temperature; SPE: solid phase extraction.

**REFERENCES**

1. Gillessen S, Attard G, Beer TM, Beltran H, Bjartell A, Bossi A, et al. Management of Patients with Advanced Prostate Cancer: Report of the Advanced Prostate Cancer Consensus Conference 2019. Eur Urol. 2020;77:508-47. doi:10.1016/j.eururo.2020.01.012.
2. Lawhn-Heath C, Salavati A, Behr SC, Rowe SP, Calais J, Fendler WP, et al. Prostate-specific Membrane Antigen PET in Prostate Cancer. Radiology. 2021;299:248-60. doi:10.1148/radiol.2021202771.
3. Afshar-Oromieh A, Haberkorn U, Schlemmer HP, Fenchel M, Eder M, Eisenhut M, et al. Comparison of PET/CT and PET/MRI hybrid systems using a 68Ga-labelled PSMA ligand for the diagnosis of recurrent prostate cancer: initial experience. Eur J Nucl Med Mol Imaging. 2014;41:887-97. doi:10.1007/s00259-013-2660-z.
4. Rowe SP, Macura KJ, Mena E, Blackford AL, Nadal R, Antonarakis ES, et al. PSMA-Based [18F]DCFPyL PET/CT Is Superior to Conventional Imaging for Lesion Detection in Patients with Metastatic Prostate Cancer. Mol Imaging Biol. 2016;18:411-9. doi:10.1007/s11307-016-0957-6.
5. Schmidkonz C, Hollweg C, Beck M, Reinfelder J, Goetz TI, Sanders JC, et al. 99mTc-MIP-1404-SPECT/CT for the detection of PSMA-positive lesions in 225 patients with biochemical recurrence of prostate cancer. Prostate. 2018;78:54-63. doi:10.1002/pros.23444.
6. Weineisen M, Schottelius M, Simecek J, Baum RP, Yildiz A, Beykan S, et al. 68Ga- and 177Lu-Labeled PSMA I&T: Optimization of a PSMA-Targeted Theranostic Concept and First Proof-of-Concept Human Studies. J Nucl Med. 2015;56:1169-76. doi: 1 0.2967/jnumed. 115.158550.
7. Benesova M, Schafer M, Bauder-Wust U, Afshar-Oromieh A, Kratochwil C, Mier W, et al. Preclinical Evaluation of a Tailor-Made DOTA-Conjugated PSMA Inhibitor with Optimized Linker Moiety for Imaging and Endoradiotherapy of Prostate Cancer. J Nucl Med. 2015;56:914-20. doi:10.2967/jnumed.114.147413.
8. Zang J, Fan X, Wang H, Liu Q, Wang J, Li H, et al. First-in-human study of 177Lu-EB-PSMA-617 in patients with metastatic castration-resistant prostate cancer. Eur J Nucl Med Mol Imaging. 2019;46:148-58. doi:10.1007/s00259-018-4096-y.
9. Kramer V, Fernandez R, Lehnert W, Jimenez-Franco LD, Soza-Ried C, Eppard E, et al. Biodistribution and dosimetry of a single dose of albumin-binding ligand [177Lu]Lu-PSMA-ALB-56 in patients with mCRPC. Eur J Nucl Med Mol Imaging. 2021;48:893-903. doi:10.1007/s00259-020-05022-3.

10. Kratochwil C, Haberkorn U, Giesel FL. 225Ac-PSMA-617 for Therapy of Prostate Cancer. Semin Nucl Med. 2020;50:133-40. doi:10.1053/j.semnuclmed.2020.02.004.

11. Khreish F, Ebert N, Ries M, Maus S, Rosar F, Bohnenberger H, et al. 225Ac-PSMA-617/177Lu-PSMA-617 tandem therapy of metastatic castration-resistant prostate cancer: pilot experience. Eur J Nucl Med Mol Imaging. 2020;47:721-8. doi:10.1007/s00259-019-04612-0.

12. Maurer T, Graefen M, van der Poel H, Hamdy F, Briganti A, Eiber M, et al. Prostate-Specific Membrane Antigen-Guided Surgery. J Nucl Med. 2020;61:6-12. doi:10.2967/jnumed.119.232330.

13. Maurer T, Robu S, Schottelius M, Schwamborn K, Rauscher I, van den Berg NS, et al. 99mTechnetium-based Prostate-specific Membrane Antigen-radioguided Surgery in Recurrent Prostate Cancer. Eur Urol. 2019;75:659-66. doi: 1 0.1 016/j.eururo.2018.03.013.

14. Schottelius M, Wirtz M, Eiber M, Maurer T, Wester HJ. [111In]PSMA-I&T: expanding the spectrum of PSMA-I&T applications towards SPECT and radioguided surgery. EJNMMI Res. 2015;5:68. doi:10.1186/s13550-015-0147-6.

15. Robu S, Schottelius M, Eiber M, Maurer T, Gschwend J, Schwaiger M, et al. Preclinical Evaluation and First Patient Application of 99mTc-PSMA-I&S for SPECT Imaging and Radioguided Surgery in Prostate Cancer. J Nucl Med. 2017;58:235-42. doi:10.2967/jnumed.116.178939.

16. Horn T, Kronke M, Rauscher I, Haller B, Robu S, Wester HJ, et al. Single Lesion on Prostate-specific Membrane Antigen-ligand Positron Emission Tomography and Low Prostate-specific Antigen Are Prognostic Factors for a Favorable Biochemical Response to Prostate-specific Membrane Antigen-targeted Radioguided Surgery in Recurrent Prostate Cancer. Eur Urol. 2019;76:517-23. doi:10.1016/j.eururo.2019.03.045.

17. Knipper S, Tilki D, Mansholt J, Berliner C, Bernreuther C, Steuber T, et al. Metastases-yield and Prostate-specific Antigen Kinetics Following Salvage Lymph Node Dissection for Prostate Cancer: A Comparison Between Conventional Surgical Approach and Prostate-specific Membrane Antigen-radioguided Surgery. Eur Urol Focus. 2019;5:50-3. doi:10.1016/j.euf.2018.09.014.

18. Rauscher I, Horn T, Eiber M, Gschwend JE, Maurer T. Novel technology of molecular radio-guidance for lymph node dissection in recurrent prostate cancer by PSMA-ligands. World J Urol. 2018;36:603-8. doi:10.1007/s00345-018-2200-3.

19. de Barros HA, van Oosterom MN, Donswijk ML, Hendrikx J, Vis AN, Maurer T, et al. Robot-assisted Prostate-specific Membrane Antigen-radioguided Salvage Surgery in Recurrent Prostate Cancer Using a DROP-IN Gamma Probe: The First Prospective Feasibility Study. Eur Urol. 2022;82:97-105. doi:10.1016/j.eururo.2022.03.002.

20. Fossati N, Suardi N, Gandaglia G, Bravi CA, Soligo M, Karnes RJ, et al. Identifying the Optimal Candidate for Salvage Lymph Node Dissection for Nodal Recurrence of Prostate Cancer: Results from a Large, Multi-institutional Analysis. Eur Urol. 2019;75:176-83. doi:10.1016/j.eururo.2018.09.009.

21. Urban S, Meyer C, Dahlbom M, Farkas I, Sipka G, Besenyi Z, et al. Radiation Dosimetry of 99mTc-PSMA I&S: A Single-Center Prospective Study. J Nucl Med. 2021;62:1075-81. doi:10.2967/jnumed.120.253476.

22. Werner P, Neumann C, Eiber M, Wester HJ, Schottelius M. [99cmTc]Tc-PSMA-I&S-SPECT/CT: experience in prostate cancer imaging in an outpatient center. EJNMMI Res. 2020;10:45. doi:10.1186/s13550-020-00635-z.

23. Wurzer A, Di Carlo D, Schmidt A, Beck R, Eiber M, Schwaiger M, et al. Radiohybrid Ligands: A Novel Tracer Concept Exemplified by 18F- or 68Ga-Labeled rhPSMA Inhibitors. J Nucl Med. 2020;61:735-42. doi:10.2967/jnumed.119.234922.

24. Wurzer A, Kunert JP, Fischer S, Felber V, Beck R, De Rose F, et al. Synthesis and Preclinical Evaluation of 177Lu-labeled Radiohybrid PSMA Ligands (rhPSMAs) for Endoradiotherapy of Prostate Cancer. J Nucl Med. 2022. doi:1121.263371.

25. Hensbergen AW, Buckle T, van Willigen DM, Schottelius M, Welling MM, van der Wijk FA, et al. Hybrid Tracers Based on Cyanine Backbones Targeting Prostate-Specific Membrane Antigen: Tuning Pharmacokinetic Properties and Exploring Dye-Protein Interaction. J Nucl Med. 2020;61:234-41. doi:10.2967/jnumed.119.233064.

26. Eder AC, Schafer M, Schmidt J, Bauder-Wust U, Roscher M, Leotta K, et al. Rational Linker Design to Accelerate Excretion and Reduce Background Uptake of Peptidomimetic PSMA-Targeting Hybrid Molecules. J Nucl Med. 2021;62:1461-7. doi:10.2967/jnumed. 120.248443.

27. Wurzer A, Parzinger M, Konrad M, Beck R, Gunther T, Felber V, et al. Preclinical comparison of four [18F, natGa]rhPSMA-7 isomers: influence of the stereoconfiguration on pharmacokinetics. EJNMMI Res. 2020;10:149. doi:10.1186/s13550-020-00740-z.

28. Weineisen M, Simecek J, Schottelius M, Schwaiger M, Wester HJ. Synthesis and preclinical evaluation of DOTAGA-conjugated PSMA ligands for functional imaging and endoradiotherapy of prostate cancer. EJNMMI Res. 2014;4:63. doi:10.1186/s13550-014-0063-1.

29. Abiraj K, Ursillo S, Tamma ML, Rylova SN, Waser B, Constable EC, et al. The tetraamine chelator outperforms HYNIC in a new technetium-99m-labelled somatostatin receptor 2 antagonist. EJNMMI Res. 2018;8:75. doi:10.1186/s13550-018-0428-y.

30. Fani M, Weingaertner V, Kolenc Peitl P, Mansi R, Gaonkar RH, Garnuszek P, et al. Selection of the First 99mTc-Labelled Somatostatin Receptor Subtype 2 Antagonist for Clinical Translation-Preclinical Assessment of Two Optimized Candidates. Pharmaceuticals (Basel). 2020;14:19. doi:10.3390/ph14010019.
31. Kubyshkin V. Experimental lipophilicity scale for coded and noncoded amino acid residues. Org Biomol Chem. 2021;19:7031-40. doi:10.1039/d1ob01213d.
32. Schottelius M, Wurzer A, Wissmiller K, Beck R, Koch M, Gorpas D, et al. Synthesis and Preclinical Characterization of the PSMA-Targeted Hybrid Tracer PSMA-I&F for Nuclear and Fluorescence Imaging of Prostate Cancer. J Nucl Med. 2019;60:71-8. doi: 10.2967/jnumed. 118.212720.
33. Benesova M, Umbricht CA, Schibli R, Muller C. Albumin-Binding PSMA Ligands: Optimization of the Tissue Distribution Profile. Mol Pharm. 2018;15:934-46. doi:1 0.1 021/acs.molpharmaceut.7b00877.
34. Mix M, Schultze-Seemann W, von Buren M, Sigle A, Omrane MA, Grabbert MT, et al. 99mTc-labelled PSMA ligand for radio-guided surgery in nodal metastatic prostate cancer: proof of principle. EJNMMI Res. 2021;11:22. doi:10.1186/s13550-021-00762-1.
35. Robu S, Schmidt A, Eiber M, Schottelius M, Gunther T, Hooshyar Yousefi B, et al. Synthesis and preclinical evaluation of novel 18F-labeled Glu-urea-Glu-based PSMA inhibitors for prostate cancer imaging: a comparison with 18F-DCFPyl and 18F-PSMA-1007. EJNMMI Res. 2018;8:30. doi:10.1186/s13550-018-0382-8.
36. lovkova L, Wangler B, Schirrmacher E, Schirrmacher R, Quandt G, Boening G, et al. para-Functionalized aryl-di-tert-butylfluorosilanes as potential labeling synthons for 18F radiopharmaceuticals. Chemistry. 2009;15:2140-7. doi:10.1002/chem.200802266.

## Claims

**1.** A compound of formula (1) or a salt thereof:

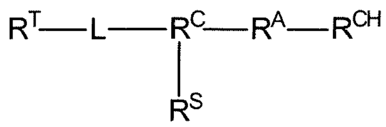

(1)

wherein

$R^T$ is a PSMA binding group;
L is a linking group;
$R^C$ is a trivalent coupling group;
$R^S$ is a silicon-containing moiety of the formula $-C(O)-R^{S3}-SiR^{S1}R^{S2}OH$, wherein $R^{S1}$ and $R^{S2}$ are independently selected from C3-C10 alkyl, and $R^{S3}$ is a group comprising a 6 membered aromatic ring;
$R^A$ is an amino acid residue; and
$R^{CH}$ is selected from

(i) a branched-chain, acyclic chelating moiety having 4 amino groups, and
(ii) a chelate moiety, wherein a radioisotope selected from $^{99m}Tc$, $^{94m}Tc$, $^{186}Re$ and $^{188}Re$ is chelated by the branched-chain, acyclic chelating moiety having 4 amino groups.

**2.** The compound or salt in accordance with claim 1, wherein $R^T$ in formula (1) is a group of the following structure:

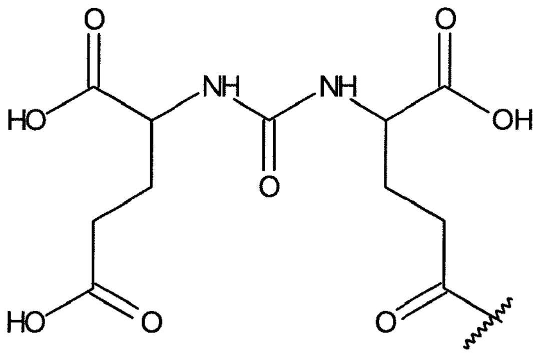

wherein the waved line marks the bond which attaches the group $R^T$ to the remainder of the compound of formula (1).

**3.** The compound or salt in accordance with claim 1 or 2, wherein $R^S$ in formula (1) is a group of the following structure (S-2):

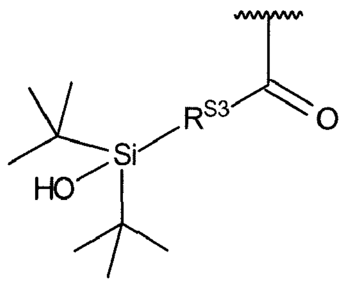

(S-2)

wherein

$R^{S3}$ is a group comprising a 6 membered aromatic ring; and
the waved line marks the bond which attaches the group $R^S$ to the remainder of the compound of formula (1).

**4.** The compound or salt in accordance with any of claims 1 to 3, wherein $R^S$ in formula (1) is a group of the following structure (S-3):

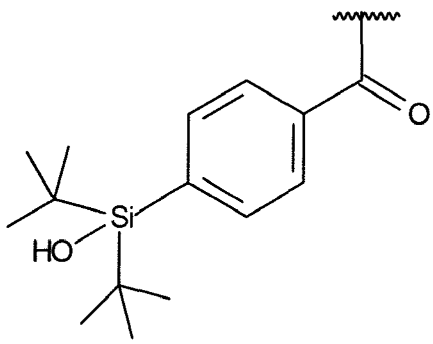

(S-3)

wherein
the waved line marks the bond which attaches the group $R^S$ to the remainder of the compound of formula (1).

**5.** The compound or salt in accordance with any of claims 1 to 4, wherein $R^A$ in formula (1) is a group of the following structure:

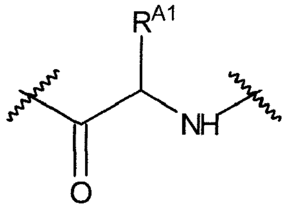

wherein

$R^{A1}$ is selected from hydrogen, $-(CH_2)_k$-COOH, $-CH_2$-Ar, and -Ar,
wherein k is 1, 2 or 3, preferably 2, and
Ar is an optionally substituted phenyl group, which may carry a substituent selected from -OH and $-NH_2$,
the waved line at the -C(O)- group shown in the formula marks a bond formed with $R^C$, and the waved line at the -NH- group shown in the formula marks a bond formed with $R^{CH}$.

6. The compound or salt in accordance with any of claims 1 to 5, wherein $R^{CH}$ in formula (1) is selected from:

(i) a N4 chelating group of the formula:

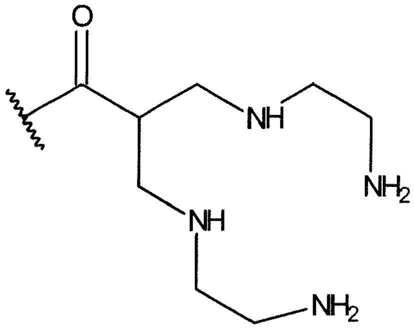

wherein
the waved line marks the bond which attaches the group $R^{CH}$ to the remainder of the compound of formula (1), and wherein optionally one or more, such as one, two or three hydrogen atoms attached to carbon atoms in the above formula can be replaced by a substituent, and
(ii) a chelate moiety, wherein a radioisotope selected from $^{99m}$Tc, $^{94m}$Tc, $^{186}$Re and $^{188}$Re is chelated by the N4 chelating group.

7. The compound or salt in accordance with any of claims 1 to 6, wherein L in formula (1) is an oligoamide residue comprising 2 to 6 subunits linked to each other via an amide bond -C(O)-NH- or an alkylated amide bond -C(O)-NR- wherein R is a C1-C6 alkyl group.

8. The compound or salt in accordance with any of claims 1 to 7, wherein $R^C$ in formula (1) is a trivalent amino acid unit which is derived from an amino acid comprising, together with the carboxylic acid group and the amino group, a side chain carrying a further functional group selected form a carboxylic acid group and an amino group.

9. The compound or salt in accordance with any of claims 1 to 8, which is a radiolabeled compound comprising a chelated radioisotope selected from $^{99m}$Tc, $^{94m}$Tc, $^{186}$Re and $^{188}$Re.

10. The compound or salt in accordance with claim 9 for use in diagnosing in *vivo* a disease which is associated with the overexpression of PSMA, wherein the disease is preferably cancer and more preferably prostate cancer and diagnosing preferably involves nuclear medicine tomography, more preferably single-photon emission computed tomography (SPECT).

11. The compound or salt in accordance with claim 9 for use in identifying *in vivo* diseased tissue which is associated with the overexpression of PSMA, wherein the diseased tissue is preferably cancer tissue and more preferably prostate cancer tissue.

**12.** The compound or salt in accordance with claim 11, wherein the diseased tissue is identified in the context of a radioguided surgery for removing the diseased tissue.

**13.** *Ex vivo* or *in vitro* use of the compound or salt in accordance with claim 9 for identifying a tissue or cell which is associated with the overexpression of PSMA, wherein the tissue or cell is preferably a cancer tissue or cell and more preferably a prostate cancer tissue or cell.

**14.** An *ex vivo* or *in vitro* method for identifying whether a tissue or cell overexpresses PSMA, comprising contacting the tissue or cell with the compound or salt in accordance with claim 9, wherein the tissue or cell is preferably a cancer tissue or cell and more preferably a prostate cancer tissue or cell.

**15.** The compound or salt in accordance with claim 9 for use in treating or preventing a disease which is associated with the overexpression of PSMA, wherein the disease is preferably cancer and more preferably prostate cancer.

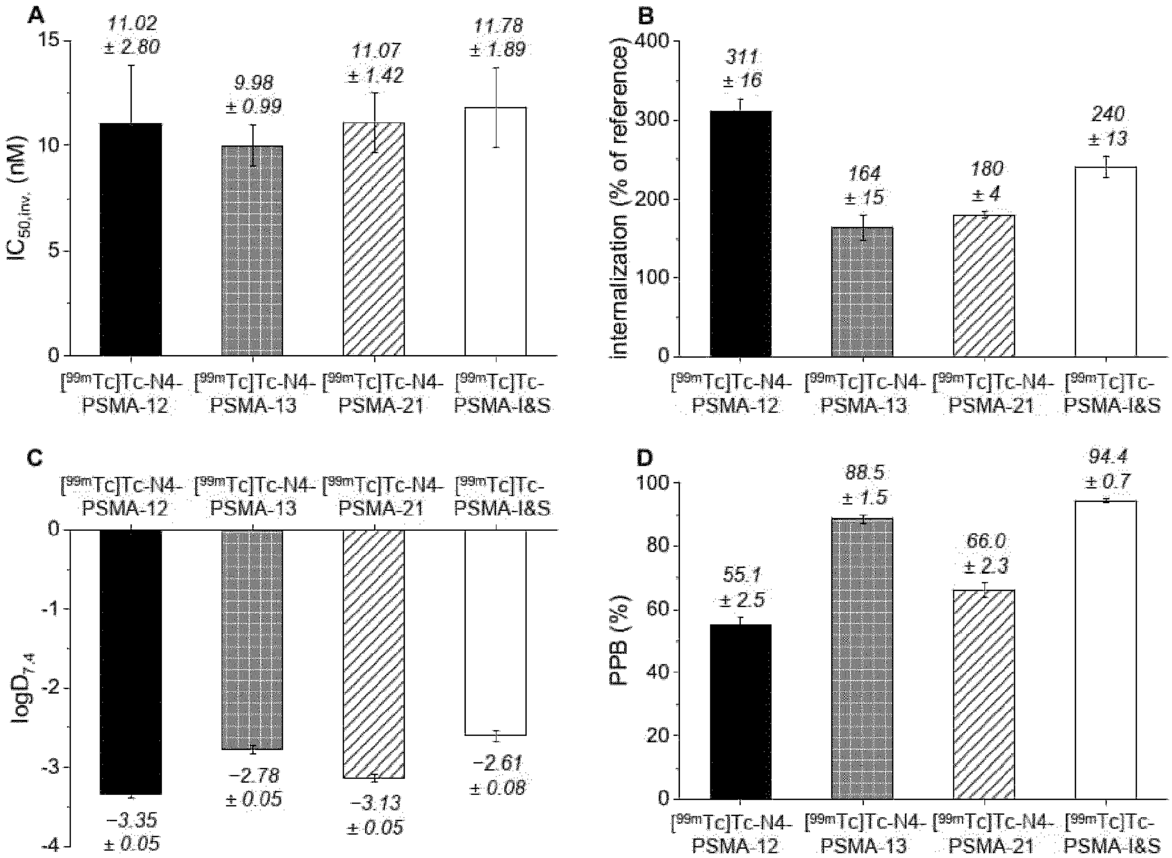
A
11.02
± 2.80
9.98
± 0.99
11.07
± 1.42
11.78
± 1.89
IC50,inv. (nM)
15
10
5
0
[99mTc]Tc-N4-PSMA-12
[99mTc]Tc-N4-PSMA-13
[99mTc]Tc-N4-PSMA-21
[99mTc]Tc-PSMA-I&S
B
311
± 16
164
± 15
180
± 4
240
± 13
internalization (% of reference)
400
300
200
100
0
[99mTc]Tc-N4-PSMA-12
[99mTc]Tc-N4-PSMA-13
[99mTc]Tc-N4-PSMA-21
[99mTc]Tc-PSMA-I&S
C
[99mTc]Tc-N4-PSMA-12
[99mTc]Tc-N4-PSMA-13
[99mTc]Tc-N4-PSMA-21
[99mTc]Tc-PSMA-I&S
logD7.4
0
-1
-2
-3
-4
−3.35
± 0.05
−2.78
± 0.05
−3.13
± 0.05
−2.61
± 0.08
D
55.1
± 2.5
88.5
± 1.5
66.0
± 2.3
94.4
± 0.7
PPB (%)
100
80
60
40
20
0
[99mTc]Tc-N4-PSMA-12
[99mTc]Tc-N4-PSMA-13
[99mTc]Tc-N4-PSMA-21
[99mTc]Tc-PSMA-I&S

Fig. 1

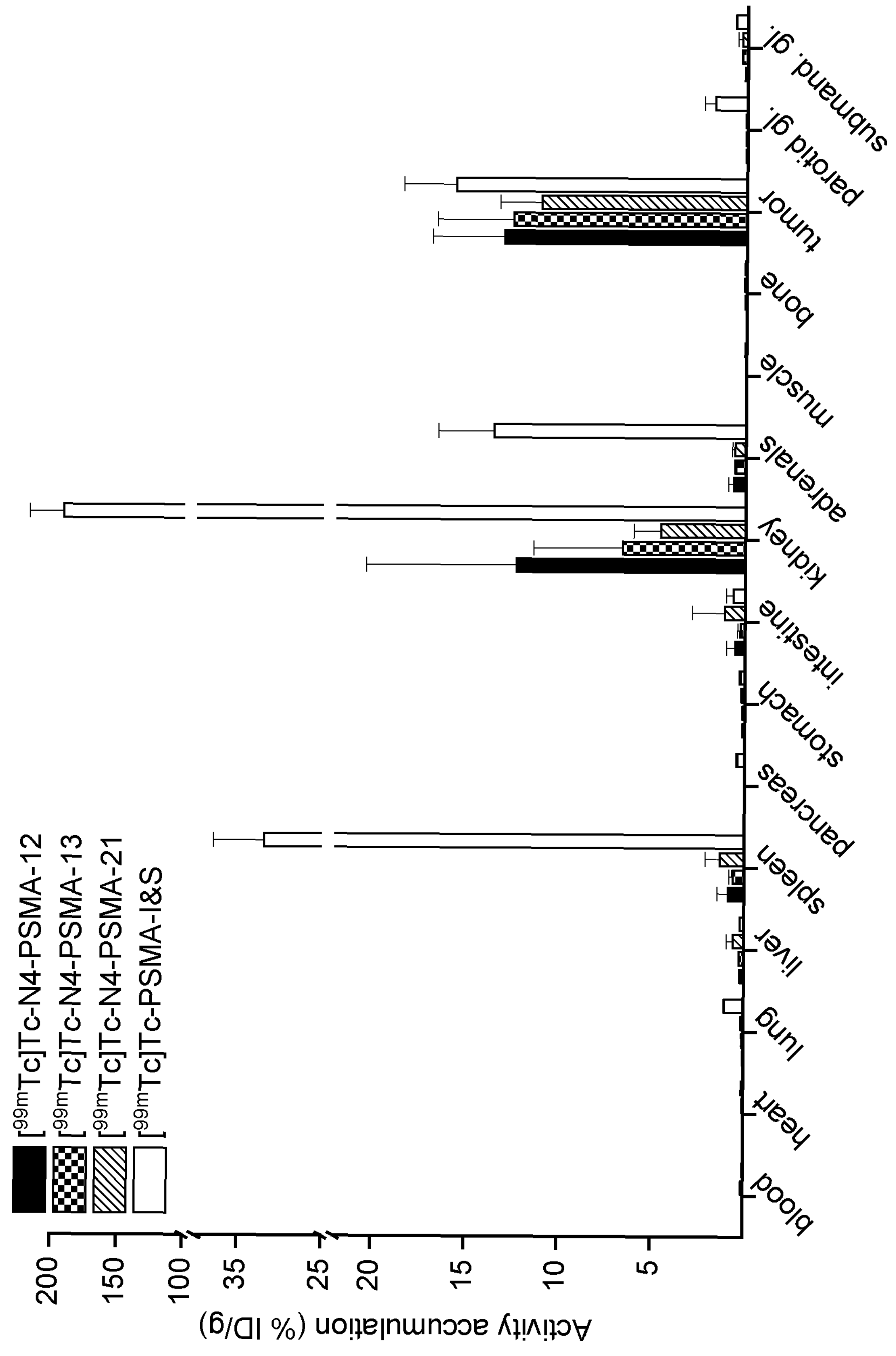
Activity accumulation (% ID/g)
200
150
100
35
25
20
15
10
5
[$^{99m}$Tc]Tc-N4-PSMA-12
[$^{99m}$Tc]Tc-N4-PSMA-13
[$^{99m}$Tc]Tc-N4-PSMA-21
[$^{99m}$Tc]Tc-PSMA-I&S
blood
heart
lung
liver
spleen
pancreas
stomach
intestine
kidney
adrenals
muscle
bone
tumor
parotid gl.
submand. gl.

Fig. 2

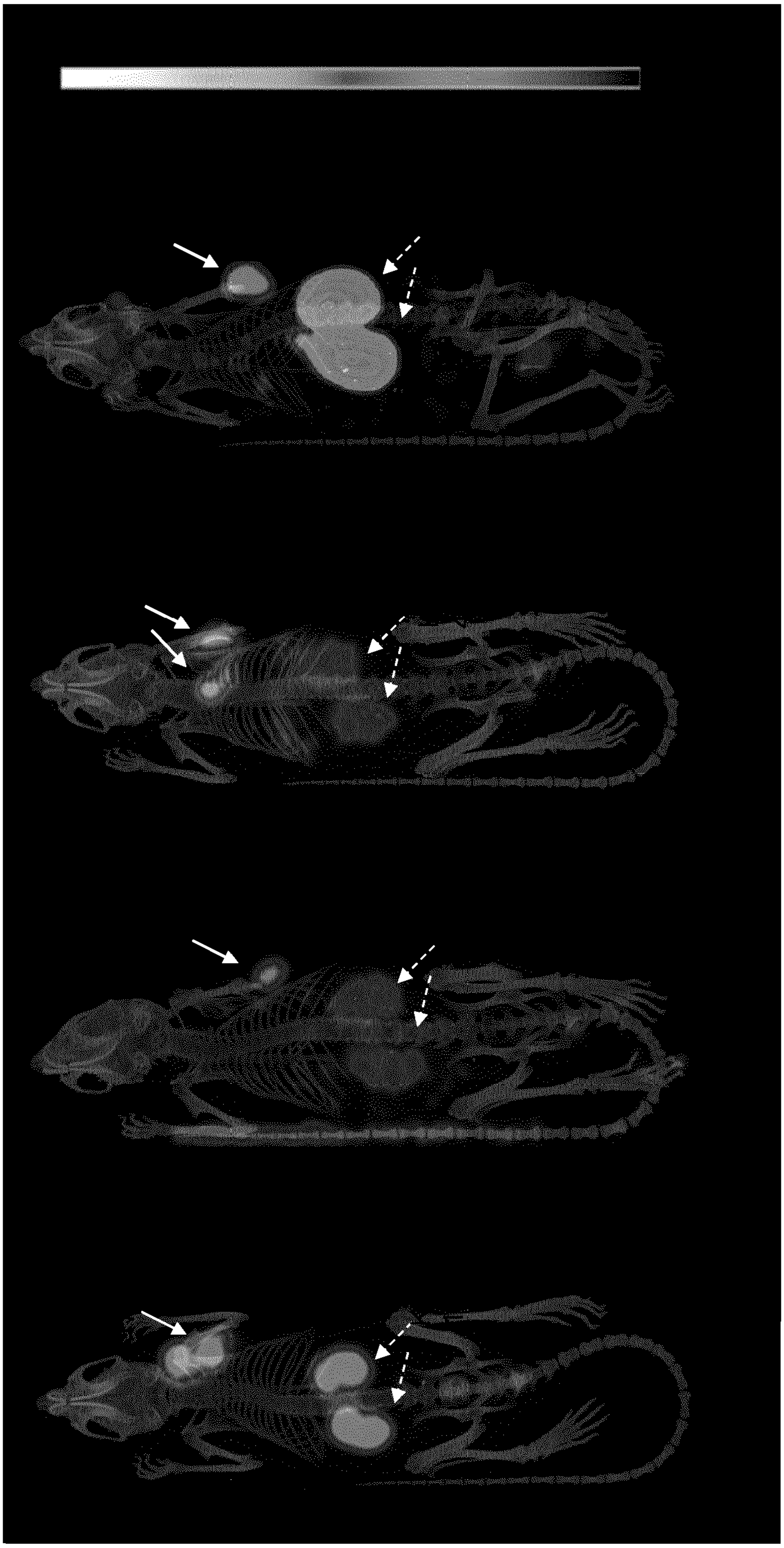

64

Fig. 3

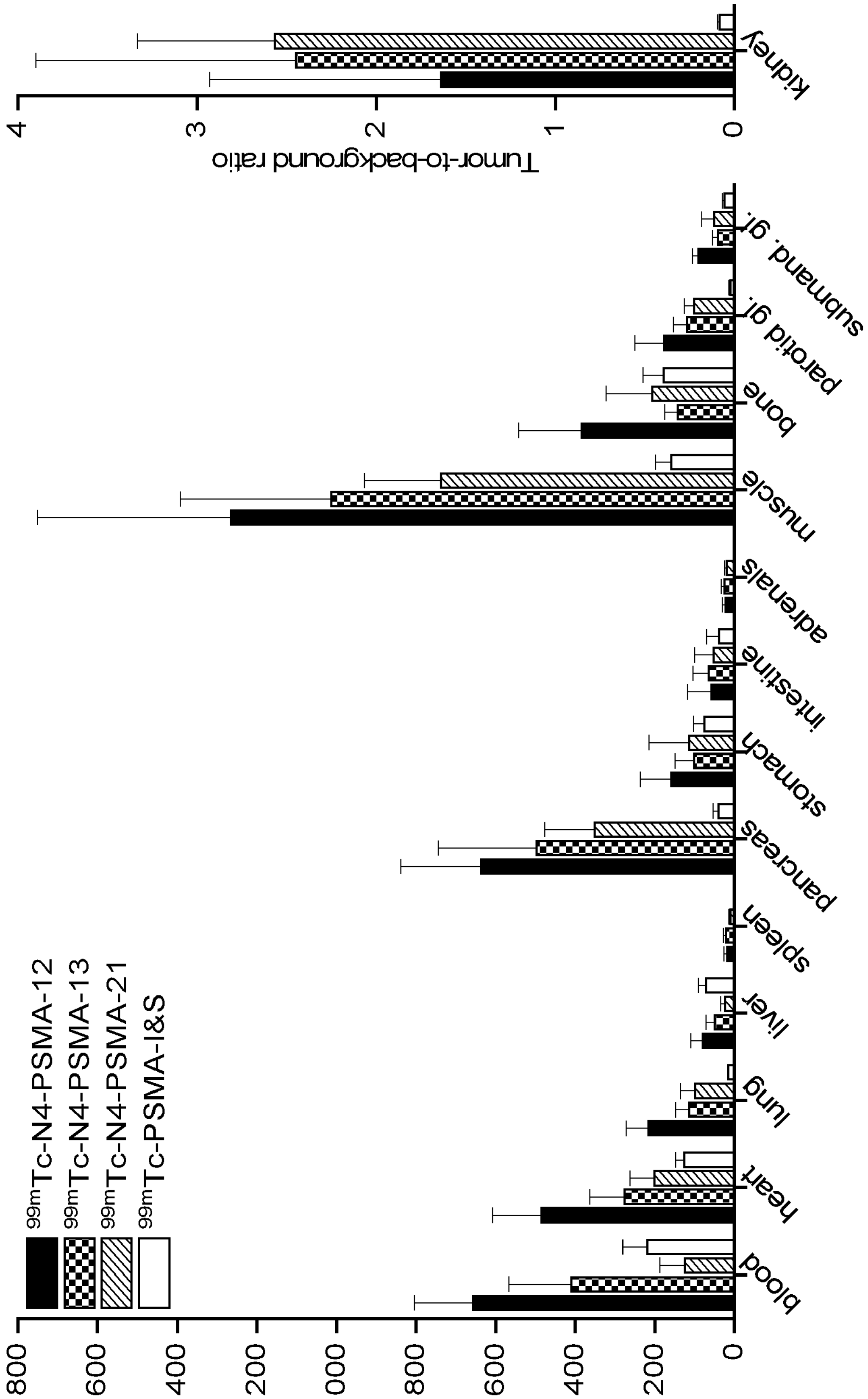
$^{99m}$Tc-N4-PSMA-12
$^{99m}$Tc-N4-PSMA-13
$^{99m}$Tc-N4-PSMA-21
$^{99m}$Tc-PSMA-I&S
Tumor-to-background ratio
0
200
400
600
800
1000
1200
1400
1600
1800
blood
heart
lung
liver
spleen
pancreas
stomach
intestine
adrenals
muscle
bone
parotid gl.
submand. gl.
Tumor-to-background ratio
0
1
2
3
4
kidney

Fig. 4

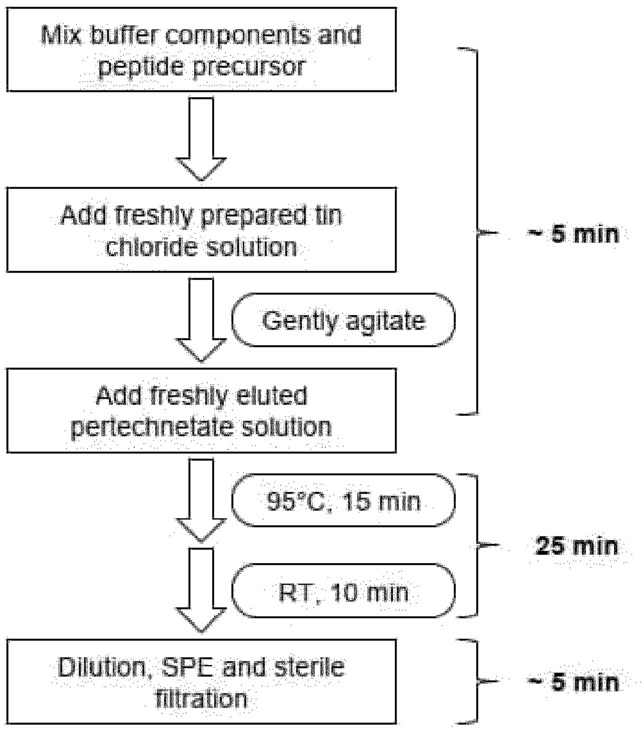
Mix buffer components and peptide precursor
Add freshly prepared tin chloride solution
Gently agitate
Add freshly eluted pertechnetate solution
95°C, 15 min
RT, 10 min
Dilution, SPE and sterile filtration
~ 5 min
25 min
~ 5 min

Fig. 5

Europäisches Patentamt
European Patent Office
Office européen des brevets

# EUROPEAN SEARCH REPORT

Application Number
EP 22 21 5651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | WO 2022/018264 A1 (UNIV MUENCHEN TECH [DE]) 27 January 2022 (2022-01-27)<br>* claims 6-8; figures 10-15; page 4, lines 20-32; page 36, line 16 to page 37, line 7; page 55, lines 14-21 and page 56, lines 32-37; page 57, line 37 to page 58, line 6; page 64, structure PSMA-7.3-SiOH * | 1-15<br>1-15 | INV.<br>A61K51/04<br>A61P35/00<br>A61K103/10 |
| Y,D | ROBU STEPHANIE ET AL: "Preclinical Evaluation and First Patient Application of 99m Tc-PSMA-I&S for SPECT Imaging and Radioguided Surgery in Prostate Cancer", THE JOURNAL OF NUCLEAR MEDICINE, vol. 58, no. 2, 15 September 2016 (2016-09-15), pages 235-242, XP055774139, US ISSN: 0161-5505, DOI: 10.2967/jnumed.116.178939 Retrieved from the Internet: URL:https://jnm.snmjournals.org/content/jnumed/58/2/235.full.pdf><br>* figure 1, Abstract * | 1-15 | TECHNICAL FIELDS SEARCHED (IPC)<br>A61K |
| Y | MICHAEL MIX ET AL: "99mTc-labelled PSMA ligand for radio-guided surgery in nodal metastatic prostate cancer: proof of principle", EJNMMI RESEARCH, BIOMED CENTRAL LTD, LONDON, UK, vol. 11, no. 1, 4 March 2021 (2021-03-04), pages 1-9, XP021287372, DOI: 10.1186/S13550-021-00762-1<br>* the whole document * | 1-15 | |

-/--

The present search report has been drawn up for all claims

| Place of search | Date of completion of the search | Examiner |
|---|---|---|
| The Hague | 4 June 2023 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

1

Europäisches Patentamt
European Patent Office
Office européen des brevets

# EUROPEAN SEARCH REPORT

Application Number
EP 22 21 5651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | ALEXANDER WURZER ET AL: "Preclinical comparison of four [F, Ga]rhPSMA-7 isomers: influence of the stereoconfiguration on pharmacokinetics", EJNMMI RESEARCH, BIOMED CENTRAL LTD, LONDON, UK, vol. 10, no. 1, 7 December 2020 (2020-12-07), pages 1-10, XP021285239, DOI: 10.1186/S13550-020-00740-Z * Abstract, results paragraph therein * ----- | 1-15 | |
| A | PIRON SARAH ET AL: "Recent advancements in 18F-labeled PSMA targeting PET radiopharmaceuticals", NUCLEAR MEDICINE AND BIOLOGY, ELSEVIER, NY, US, vol. 106, 30 December 2021 (2021-12-30), pages 29-51, XP086988375, ISSN: 0969-8051, DOI: 10.1016/J.NUCMEDBIO.2021.12.005 [retrieved on 2021-12-30] * paragraph 4.3.1.1 * ----- | 1-15 | TECHNICAL FIELDS SEARCHED (IPC) |
| A | LANGBEIN THOMAS ET AL: "Utility of 18 F-rhPSMA-7.3 PET for Imaging of Primary Prostate Cancer and Preoperative Efficacy in N-Staging of Unfavorable Intermediate- to Very High-Risk Patients Validated by Histopathology", THE JOURNAL OF NUCLEAR MEDICINE, vol. 63, no. 9, 6 January 2022 (2022-01-06), pages 1334-1342, XP093051586, US ISSN: 0161-5505, DOI: 10.2967/jnumed.121.263440 * abstract * ----- -/-- | 1-15 | |

The present search report has been drawn up for all claims

1

| Place of search | Date of completion of the search | Examiner |
|---|---|---|
| The Hague | 4 June 2023 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 22 21 5651

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | S. M. HILLIER ET AL: "99mTc-Labeled Small-Molecule Inhibitors of Prostate-Specific Membrane Antigen for Molecular Imaging of Prostate Cancer", THE JOURNAL OF NUCLEAR MEDICINE, vol. 54, no. 8, 1 August 2013 (2013-08-01), pages 1369-1376, XP055280729, US ISSN: 0161-5505, DOI: 10.2967/jnumed.112.116624 * the whole document * ----- | 1-15 | |

TECHNICAL FIELDS SEARCHED (IPC)

The present search report has been drawn up for all claims

1

| Place of search | Date of completion of the search | Examiner |
|---|---|---|
| The Hague | 4 June 2023 | Burema, Shiri |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

## ANNEX TO THE EUROPEAN SEARCH REPORT ON EUROPEAN PATENT APPLICATION NO.

EP 22 21 5651

This annex lists the patent family members relating to the patent documents cited in the above-mentioned European search report. The members are as contained in the European Patent Office EDP file on
The European Patent Office is in no way liable for these particulars which are merely given for the purpose of information.

04-06-2023

| Patent document cited in search report | Publication date | Patent family member(s) | Publication date |
|---|---|---|---|
| WO 2022018264 A1 | 27-01-2022 | EP 4185336 A1 | 31-05-2023 |
| | | WO 2022018264 A1 | 27-01-2022 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Non-patent literature cited in the description


- **VALKO, K. ; NUNHUCK, S. ; BEVAN, C. ; ABRAHAM, M. H. ; REYNOLDS, D. P.** Fast gradient HPLC method to determine compounds binding to human serum albumin. Relationships with octanol/water and immobilized artificial membrane lipophilicity. *J Pharm Sci.,* 2003, vol. 92, 2236-2248
- **VALKO, K. ; NUNHUCK, S. ; BEVAN, C. ; ABRAHAM, M. H. ; REYNOLDS, D. P.** *J Pharm Sci.,* 2003, vol. 92, 2236-2248
- **YAMAZAKI, K. ; KANAOKA, M.** Computational prediction of the plasma protein-binding percent of diverse pharmaceutical compounds. *J Pharm Sci.,* 2004, vol. 93, 1480-1494
- **GILLESSEN S ; ATTARD G ; BEER TM ; BELTRAN H ; BJARTELL A ; BOSSI A et al.** Management of Patients with Advanced Prostate Cancer: Report of the Advanced Prostate Cancer Consensus Conference 2019. *Eur Urol.,* 2020, vol. 77, 508-47
- **LAWHN-HEATH C ; SALAVATI A ; BEHR SC ; ROWE SP ; CALAIS J ; FENDLER WP et al.** Prostate-specific Membrane Antigen PET in Prostate Cancer. *Radiology.,* 2021, vol. 299, 248-60
- **AFSHAR-OROMIEH A ; HABERKORN U ; SCHLEMMER HP ; FENCHEL M ; EDER M ; EISENHUT M et al.** Comparison of PET/CT and PET/MRI hybrid systems using a Ga-labelled PSMA ligand for the diagnosis of recurrent prostate cancer: initial experience. *Eur J Nucl Med Mol Imaging.,* 2014, vol. 41, 887-97
- **ROWE SP ; MACURA KJ ; MENA E ; BLACKFORD AL ; NADAL R ; ANTONARAKIS ES et al.** PSMA-Based [18F]DCFPyL PET/CT Is Superior to Conventional Imaging for Lesion Detection in Patients with Metastatic Prostate Cancer. *Mol Imaging Biol.,* 2016, vol. 18, 411-9
- **SCHMIDKONZ C ; HOLLWEG C ; BECK M ; REINFELDER J ; GOETZ TL ; SANDERS JC et al.** Tc-MIP-1404-SPECT/CT for the detection of PSMA-positive lesions in 225 patients with biochemical recurrence of prostate cancer. *Prostate.,* 2018, vol. 78, 54-63
- **WEINEISEN M ; SCHOTTELIUS M ; SIMECEK J ; BAUM RP ; YILDIZ A ; BEYKAN S et al.** Ga- and Lu-Labeled PSMA I&T: Optimization of a PSMA-Targeted Theranostic Concept and First Proof-of-Concept Human Studies. *J Nucl Med.,* 2015, vol. 56, 1169-76
- **BENESOVA M ; SCHAFER M ; BAUDER-WUST U ; AFSHAR-OROMIEH A ; KRATOCHWIL C ; MIER W et al.** Preclinical Evaluation of a Tailor-Made DOTA-Conjugated PSMA Inhibitor with Optimized Linker Moiety for Imaging and Endoradiotherapy of Prostate Cancer. *J Nucl Med.,* 2015, vol. 56, 914-20
- **ZANG J ; FAN X ; WANG H ; LIU Q ; WANG J ; LI H et al.** First-in-human study of Lu-EB-PSMA-617 in patients with metastatic castration-resistant prostate cancer. *Eur J Nucl Med Mol Imaging.,* 2019, vol. 46, 148-58
- **KRAMER V ; FERNANDEZ R ; LEHNERT W ; JIMENEZ-FRANCO LD ; SOZA-RIED C ; EPPARD E et al.** Biodistribution and dosimetry of a single dose of albumin-binding ligand [177Lu]Lu-PSMA-ALB-56 in patients with mCRPC. *Eur J Nucl Med Mol Imaging.,* 2021, vol. 48, 893-903
- **KRATOCHWIL C ; HABERKORN U ; GIESEL FL.** Ac-PSMA-617 for Therapy of Prostate Cancer. *Semin Nucl Med.,* 2020, vol. 50, 133-40
- **KHREISH F ; EBERT N ; RIES M ; MAUS S ; ROSAR F ; BOHNENBERGER H et al.** Ac-PSMA-617/177Lu-PSMA-617 tandem therapy of metastatic castration-resistant prostate cancer: pilot experience. *Eur J Nucl Med Mol Imaging.,* 2020, vol. 47, 721-8
- **MAURER T ; GRAEFEN M ; VAN DER POEL H ; HAMDY F ; BRIGANTI A ; EIBER M et al.** Prostate-Specific Membrane Antigen-Guided Surgery. *J Nucl Med.,* 2020, vol. 61, 6-12
- **MAURER T ; ROBU S ; SCHOTTELIUS M ; SCHWAMBORN K ; RAUSCHER I ; VAN DEN BERG NS et al.** Technetium-based Prostate-specific Membrane Antigen-radioguided Surgery in Recurrent Prostate Cancer. *Eur Urol.,* 2019, vol. 75, 659-66
- **SCHOTTELIUS M ; WIRTZ M ; EIBER M ; MAURER T ; WESTER HJ.** In]PSMA-I&T: expanding the spectrum of PSMA-I&T applications towards SPECT and radioguided surgery. *EJNMMI Res,* 2015, vol. 5, 68
- **ROBU S ; SCHOTTELIUS M ; EIBER M ; MAURER T ; GSCHWEND J ; SCHWAIGER M et al.** Preclinical Evaluation and First Patient Application of Tc-PSMA-I&S for SPECT Imaging and Radioguided Surgery in Prostate Cancer. *J Nucl Med.,* 2017, vol. 58, 235-42

- **HORN T ; KRONKE M ; RAUSCHER I ; HALLER B ; ROBU S ; WESTER HJ et al.** Single Lesion on Prostate-specific Membrane Antigen-ligand Positron Emission Tomography and Low Prostate-specific Antigen Are Prognostic Factors for a Favorable Biochemical Response to Prostate-specific Membrane Antigen-targeted Radioguided Surgery in Recurrent Prostate Cancer. *Eur Urol.,* 2019, vol. 76, 517-23
- **KNIPPER S ; TILKI D ; MANSHOLT J ; BERLINER C ; BERNREUTHER C ; STEUBER T et al.** Metastases-yield and Prostate-specific Antigen Kinetics Following Salvage Lymph Node Dissection for Prostate Cancer: A Comparison Between Conventional Surgical Approach and Prostate-specific Membrane Antigen-radioguided Surgery. *Eur Urol Focus.,* 2019, vol. 5, 50-3
- **RAUSCHER I ; HORN T ; EIBER M ; GSCHWEND JE ; MAURER T.** Novel technology of molecular radio-guidance for lymph node dissection in recurrent prostate cancer by PSMA-ligands. *World J Urol.,* 2018, vol. 36, 603-8
- **DE BARROS HA ; VAN OOSTEROM MN ; DONSWIJK ML ; HENDRIKX J ; VIS AN ; MAURER T et al.** Robot-assisted Prostate-specific Membrane Antigen-radioguided Salvage Surgery in Recurrent Prostate Cancer Using a DROP-IN Gamma Probe: The First Prospective Feasibility Study. *Eur Urol.,* 2022, vol. 82, 97-105
- **FOSSATI N ; SUARDI N ; GANDAGLIA G ; BRAVI CA ; SOLIGO M ; KARNES RJ et al.** Identifying the Optimal Candidate for Salvage Lymph Node Dissection for Nodal Recurrence of Prostate Cancer: Results from a Large, Multi-institutional Analysis. *Eur Urol.,* 2019, vol. 75, 176-83
- **URBAN S ; MEYER C ; DAHLBOM M ; FARKAS I ; SIPKA G ; BESENYI Z et al.** Radiation Dosimetry of Tc-PSMA I&S: A Single-Center Prospective Study. *J Nucl Med.,* 2021, vol. 62, 1075-81
- **WERNER P ; NEUMANN C ; EIBER M ; WESTER HJ ; SCHOTTELIUS M.** Tc]Tc-PSMA-I&S-SPECT/CT: experience in prostate cancer imaging in an outpatient center. *EJNMMI Res,* 2020, vol. 10, 45
- **WURZER A ; DI CARLO D ; SCHMIDT A ; BECK R ; EIBER M ; SCHWAIGER M et al.** Radiohybrid Ligands: A Novel Tracer Concept Exemplified by F- or Ga-Labeled rhPSMA Inhibitors. *J Nucl Med.,* 2020, vol. 61, 735-42
- **WURZER A ; KUNERT JP ; FISCHER S ; FELBER V ; BECK R ; DE ROSE F et al.** Synthesis and Preclinical Evaluation of Lu-labeled Radiohybrid PSMA Ligands (rhPSMAs) for Endoradiotherapy of Prostate Cancer. *J Nucl Med.,* 2022
- **HENSBERGEN AW ; BUCKLE T ; VAN WILLIGEN DM ; SCHOTTELIUS M ; WELLING MM ; VAN DER WIJK FA et al.** Hybrid Tracers Based on Cyanine Backbones Targeting Prostate-Specific Membrane Antigen: Tuning Pharmacokinetic Properties and Exploring Dye-Protein Interaction. *J Nucl Med.,* 2020, vol. 61, 234-41
- **EDER AC ; SCHAFER M ; SCHMIDT J ; BAUDER-WUST U ; ROSCHER M ; LEOTTA K et al.** Rational Linker Design to Accelerate Excretion and Reduce Background Uptake of Peptidomimetic PSMA-Targeting Hybrid Molecules. *J Nucl Med.,* 2021, vol. 62, 1461-7
- **WURZER A ; PARZINGER M ; KONRAD M ; BECK R ; GUNTHER T ; FELBER V et al.** Preclinical comparison of four [18F, Ga]rhPSMA-7 isomers: influence of the stereoconfiguration on pharmacokinetics. *EJNMMI Res.,* 2020, vol. 10, 149
- **WEINEISEN M ; SIMECEK J ; SCHOTTELIUS M ; SCHWAIGER M ; WESTER HJ.** Synthesis and preclinical evaluation of DOTAGA-conjugated PSMA ligands for functional imaging and endoradiotherapy of prostate cancer. *EJNMMI Res,* 2014, vol. 4, 63
- **ABIRAJ K ; URSILLO S ; TAMMA ML ; RYLOVA SN ; WASER B ; CONSTABLE EC et al.** The tetraamine chelator outperforms HYNIC in a new technetium-99m-labelled somatostatin receptor 2 antagonist. *EJNMMI Res.,* 2018, vol. 8, 75
- **FANI M ; WEINGAERTNER V ; KOLENC PEITL P ; MANSI R ; GAONKAR RH ; GARNUSZEK P et al.** Selection of the First Tc-Labelled Somatostatin Receptor Subtype 2 Antagonist for Clinical Translation-Preclinical Assessment of Two Optimized Candidates. *Pharmaceuticals (Basel).,* 2020, vol. 14, 19
- **KUBYSHKIN V.** Experimental lipophilicity scale for coded and noncoded amino acid residues. *Org Biomol Chem.,* 2021, vol. 19, 7031-40
- **SCHOTTELIUS M ; WURZER A ; WISSMILLER K ; BECK R ; KOCH M ; GORPAS D et al.** Synthesis and Preclinical Characterization of the PSMA-Targeted Hybrid Tracer PSMA-I&F for Nuclear and Fluorescence Imaging of Prostate Cancer. *J Nucl Med.,* 2019, vol. 60, 71-8
- **BENESOVA M ; UMBRICHT CA ; SCHIBLI R ; MULLER C.** Albumin-Binding PSMA Ligands: Optimization of the Tissue Distribution Profile. *Mol Pharm.,* 2018, vol. 15, 934-46
- **MIX M ; SCHULTZE-SEEMANN W ; VON BUREN M ; SIGLE A ; OMRANE MA ; GRABBERT MT et al.** Tc-labelled PSMA ligand for radio-guided surgery in nodal metastatic prostate cancer: proof of principle. *EJNMMI Res.,* 2021, vol. 11, 22

- **ROBU S ; SCHMIDT A ; EIBER M ; SCHOTTELIUS M ; GUNTHER T ; HOOSHYAR YOUSEFI B et al.** Synthesis and preclinical evaluation of novel F-labeled Glu-urea-Glu-based PSMA inhibitors for prostate cancer imaging: a comparison with F-DCFPyl and F-PSMA-1007. *EJNMMI Res.,* 2018, vol. 8, 30
- **LOVKOVA L ; WANGLER B ; SCHIRRMACHER E ; SCHIRRMACHER R ; QUANDT G ; BOENING G et al.** para-Functionalized aryl-di-tert-butylfluorosilanes as potential labeling synthons for F radiopharmaceuticals. *Chemistry.,* 2009, vol. 15, 2140-7